(19) Europäisches Patentamt
European Patent Office
Office européen des brevets

(11) EP 0 667 379 B1

(12) # EUROPÄISCHE PATENTSCHRIFT

(45) Veröffentlichungstag und Bekanntmachung des Hinweises auf die Patenterteilung:
**17.07.2002 Patentblatt 2002/29**

(51) Int Cl.[7]: **C09D 7/12**, C07C 49/84, C07C 45/46, C07C 45/71, C07C 45/69, C07C 323/22, C07F 7/08

(21) Anmeldenummer: **95810065.3**

(22) Anmeldetag: **01.02.1995**

(54) **Holzschutzanstrich**

Wood protecting paint

Peinture de protection du bois

(84) Benannte Vertragsstaaten:
**BE DE ES FR GB IT NL SE**

(30) Priorität: **10.02.1994 CH 40594**

(43) Veröffentlichungstag der Anmeldung:
**16.08.1995 Patentblatt 1995/33**

(73) Patentinhaber: **Ciba Specialty Chemicals Holding Inc.**
**4057 Basel (CH)**

(72) Erfinder:
- **Valet, Andreas, Dr.**
  **D-79589 Binzen (DE)**
- **Meuwly, Roger, Dr.**
  **CH-1784 Cournillens (CH)**

(56) Entgegenhaltungen:
**GB-A- 810 570**
**US-A- 4 051 161**
**US-A- 4 374 674**
**US-A- 4 495 360**

- **DATABASE WPI Section Ch, Week 9001 Derwent Publications Ltd., London, GB; Class A82, AN 90-002898 XP002018784 & JP-A-01 284 368 (EARTH SEIYAKU KK) , 15.November 1989**
- **DATABASE WPI Section Ch, Week 9244 Derwent Publications Ltd., London, GB; Class A32, AN 92-360024 XP002018785 & JP-A-04 259 505 (MATSUSHITA ELECTRIC WORKS LTD) , 16.September 1992**
- **DATABASE WPI Section Ch, Week 8509 Derwent Publications Ltd., London, GB; Class A97, AN 85-052258 XP002018786 & JP-A-60 008 004 (OTSUKA KAGU KOGYO) , 16.Januar 1985**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 667 379 B1

Printed by Jouve, 75001 PARIS (FR)

## Beschreibung

Gegenstand der Erfindung ist ein Verfahren zum Schützen von Holzoberflächen gegen Schädigung durch Licht sowie ein Schutzanstrich für Holz.

Holzoberflächen, die intensivem Sonnenlicht ausgesetzt sind, werden vor allem durch das im Sonnenlicht enthaltene UV-Licht geschädigt. Die polymeren Bestandteile des Holzes werden abgebaut, dadurch kommt es zu einer Aufrauhung und Verfärbung der Oberfläche. In der Folge tritt als weitere Schädigung ein Befall durch Mikrooganismen, insbesondere durch Pilze, ein.

Die übliche Methode, Holz gegen Lichtschädigung zu schützen, ohne auf das optische Bild der Holzoberfläche zu verzichten, ist das Ueberziehen mit einem farblosen Lack, der ein Lichtschutzmittel, insbesondere einen UV-Absorber, enthält.

Es wurde nun gefunden, dass ausgewählte UV-Absorber aus der Reihe der Benzophenone eine deutliche Stabilisatorwirkung gegen den Einfluss von Licht zeigen, wenn sie in einer Anstrichzusammensetzung appliziert werden.

Gegenstand der Erfindung ist somit ein Verfahren zum Schützen von Holzoberflächen gegen Schädigung durch Licht durch Behandlung mit einer Anstrichzusammensetzung, enthaltend ein Benzophenon der Formel I

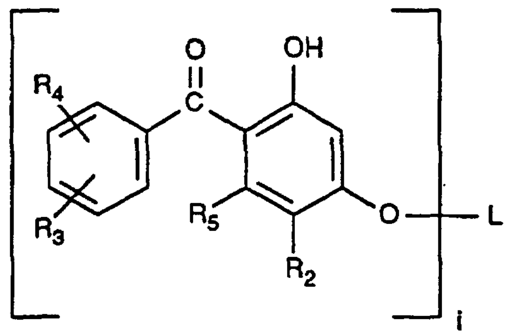 (I),

worin

i eine Zahl von eins bis vier,

L, wenn i eins ist, H, $C_1$-$C_{20}$-Alkyl, mit ein oder zwei -OH, mit Carboxyl, mit $C_1$-$C_{12}$-Alkoxy, mit Phenoxy, mit $C_2$-$C_{20}$-Alkylcarbonyl, mit $C_3$-$C_{20}$-Alkenylcarbonyl, mit $C_2$-$C_{20}$-Alkanoyloxy, mit $C_3$-$C_{20}$-Alkenoyloxy oder mit Phenyl substituiertes $C_2$-$C_{20}$-Alkyl, Glycidyl, mit ein bis sechs O-Atomen unterbrochenes oder mit ein oder zwei Carbonyloxy- oder Oxycarbonyl-Gruppen unterbrochenes $C_4$-$C_{20}$-Alkyl, $C_2$-$C_{18}$-Alkenyl, $C_2$-$C_{18}$-Alkanoyl, $C_3$-$C_{18}$-Alkenoyl, Benzoyl, mit ein oder zwei $C_1$-$C_4$-Alkyl-Gruppen substituiertes Benzoyl, gegebenenfalls substituiertes Phenyl oder Naphthyl oder eine Gruppe der Formel II, III, IV, V oder VI

$-(CH_2)_j-CH(OH)-(CH_2)_k-O-R_9$ (II) mit j, k = 0-4;

$-(CH_2)_m-COOR_9$ (III) mit m = 1-4;

wobei $R_9$ $C_1$-$C_{18}$-Alkyl oder Phenyl ist;

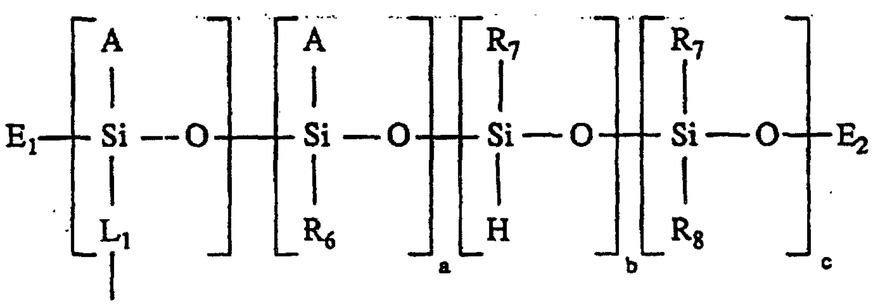 (IV);

wobei a = 0-50, b = 0-50 und c = 0-50 ist;

A $R_7$ oder $-O-Si(R_7)_3$;

$E_1$ OH, $C_1$-$C_{12}$-Alkyl, $C_1$-$C_4$-Alkoxy, Cyclohexyl, Phenyl oder $-O-Si(R_7)_3$;

$E_2$ H, $C_1$-$C_{12}$-Alkyl, Cyclohexyl, Phenyl, -Si$(R_7)_3$ oder -Si$(R_6)(R_7)_2$;
wobei $E_1$ und $E_2$ zusammen auch eine direkte Bindung bedeuten können;
$L_1$ eine direkte Bindung oder eine zweiwertige Gruppe der Formeln $-C_nH_{2n}-$, $-(CH_2)_n-O-$, $-CH_2CH(OH)CH_2-O-$ oder $-CH_2CH(OH)CH_2-O-(CH_2)_3-$, mit n = 1-4:
$R_6$ ein Rest der Formel

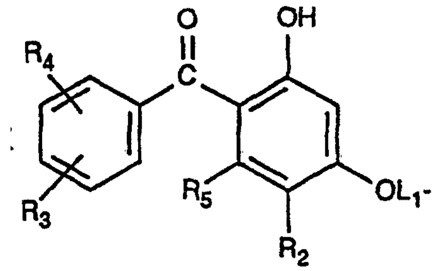

$R_7$ $C_1$-$C_{12}$-Alkyl, $C_1$-$C_4$-Alkoxy, Cyclohexyl oder Phenyl; und
$R_8$ $C_1$-$C_{18}$-Alkyl, $C_1$-$C_4$-Alkoxy, $C_5$-$C_7$-Cycloalkyl oder Phenyl ist;

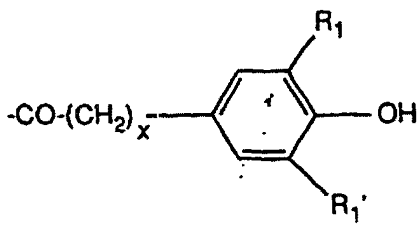 (V)

wobei x 0, 1 oder 2 ist;
$R_1$ $C_1$-$C_{12}$-Alkyl oder $C_5$-$C_8$-Cycloalkyl; und
$R_1'$ sekundäres oder tertiäres $C_3$-$C_{12}$-Alkyl oder $C_5$-$C_8$-Cycloalkyl ist;

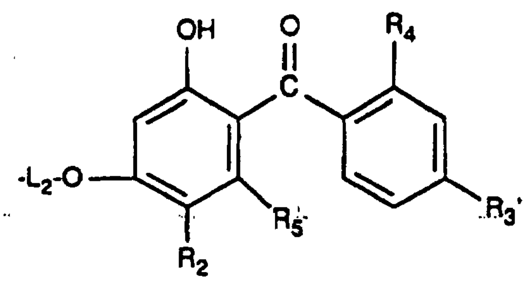 (VI)

wobei $L_2$ die Bedeutung von L, wenn i zwei ist, hat; und
$R_3'$ H oder $C_1$-$C_{18}$-Alkyl ist;

L, wenn i zwei ist, $C_1$-$C_{12}$-Alkylen, mit -OH, mit $C_1$-$C_8$-Alkoxy, mit $C_2$-$C_{20}$-Alkoxycarbonyl, mit $C_2$-$C_{20}$-Alkanoyloxy, mit $C_3$-$C_{20}$-Alkenoyloxy oder mit einer Silyl-Gruppe der Formel (IV) substituiertes $C_3$-$C_{12}$-Alkylen, mit ein bis sechs O-Atomen unterbrochenes oder mit ein oder zwei Carbonyloxy- oder Oxycarbonyl-Gruppen unterbrochenes $C_4$-$C_{20}$-Alkylen, o-Xylylen, m-Xylylen, p-Xylylen, iso-Phthaloyl, Phthaloyl, Terephthaloyl oder $\alpha,\omega$-$C_4$-$C_{12}$-Alkandioyl;
L, wenn i drei ist, $C_3$-$C_{12}$-Alkantriyl, $C_3$-$C_{12}$-Alkantrioyl, Trimellitoyl oder mit drei Carbonyloxy- oder Oxycarbonyl-Gruppen unterbrochenes $C_6$-$C_{20}$-Alkantriyl;
L, wenn i vier ist, $C_4$-$C_{16}$-Alkantetrayl, $C_4$-$C_{16}$-Alkantetroyl, Pyromellitoyl oder mit vier Carbonyloxy- oder Oxycarbonyl-Gruppen unterbrochenes $C_8$-$C_{24}$-Alkantetrayl;
$R_2$ H, $C_1$-$C_{18}$-Alkyl oder Cl;

$R_3$ $-SR_{10}$, $-SOR_{10}$ oder $-SO_2R_{10}$;
$R_4$ H, -OH, $C_1$-$C_{18}$-Alkyl oder $C_2$-$C_{18}$-Alkenyl;
$R_5$ H, -OL, mit L in der Bedeutung wenn i eins ist, oder Cl; und
$R_{10}$ $C_1$-$C_{20}$-Alkyl, mit -OH, mit $C_1$-$C_{12}$-Alkoxy, mit einer Silyl-Gruppe der Formel (IV), mit $C_2$-$C_{12}$-Alkanoyloxy, mit $C_3$-$C_{12}$-Alkenoyloxy oder mit Halogen substituiertes $C_2$-$C_{20}$-Alkyl, $C_3$-$C_{18}$-Alkenyl, $C_5$-$C_{12}$-Cycloalkyl, $C_7$-$C_{15}$-Phenylalkyl, $C_6$-$C_{10}$-Aryl, mit ein oder zwei $C_1$-$C_4$-Alkyl-Gruppen substituiertes $C_6$-$C_{10}$-Aryl oder 1,1,2,2-Tetrahydroperfluoro-$C_6$-$C_{16}$-alkyl;

bedeutet.

Bedeuten Substituenten Alkyl mit bis zu 20 Kohlenstoffatomen, so kommen hierfür die Reste wie Methyl, Ethyl, Propyl, Butyl, Pentyl, Hexyl, Octyl, Decyl, Dodecyl, Tetradecyl, Hexadecyl, Octadecyl und Eicosyl sowie entsprechende verzweigte Isomere in Frage.

Bedeuten Substituenten Alkenyl mit bis zu 18 Kohlenstoffatomen, so kommen hierfür die Reste wie 1-Propenyl, Allyl, Methallyl, 2-Butenyl, 2-Pentenyl, 2-Hexenyl, 2-Octenyl, 4-tert.-Butyl-2-butenyl oder von obigen Alkylresten abgeleitete Reste in Frage.

Bedeuten Substituenten Alkoxy mit bis zu 12 Kohlenstoffatomen, so kommen hierfür die Reste wie Methoxy, Ethoxy, Propoxy oder Butoxy sowie entsprechende verzweigte Isomere oder von obigen Alkylresten abgeleitete Reste in Frage.

Bedeuten Substituenten Alkanoyl, so kommen hierfür die Reste wie Acetyl, Propionyl, Butyryl, Capryl oder Lauroyl sowie entsprechende verzweigte Isomere oder von obigen Alkylresten abgeleitete Reste in Frage.

Bedeuten Substituenten Alkantriyl, so kommen hierfür die Reste wie 1,2,3-Propantriyl, 1,2,2-Neopentantriyl oder 1,2,2-Neohexantriyl sowie entsprechende verzweigte Isomere oder von obigen Alkylresten abgeleitete Reste in Frage.

Bedeuten Substituenten Alkantetrayl, so kommen hierfür die Reste wie Pentaerythrityl oder 1,2,3,4-Butantetrayl sowie entsprechende verzweigte Isomere oder von obigen Alkylresten abgeleitete Reste in Frage.

Bedeuten Substituenten Cycloalkyl, so kommen hierfür die Reste wie Cyclopentyl, Cyclohexyl, Cycloheptyl, Cyclooctyl oder Cyclododecyl in Frage.

Bedeuten Substituenten Phenylalkyl, so kommen hierfür die Reste wie Benzyl, Phenethyl, $\alpha$-Methylbenzyl oder $\alpha,\alpha$-Dimethylbenzyl in Frage.

Bedeuten Substituenten Aryl, so kommen hierfür die Reste wie Phenyl oder Naphthyl in Frage.

Bedeuten Substituenten Alkylen, so kommen hierfür die Reste wie Ethylen, Tetramethylen, Hexamethylen, 2-Methyl-1,4-Tetramethylen, 2,2-Dimethyl-1,3-trimethylen, Octamethylen, Decamethylen oder Dodecamethylen in Frage.

Bedeuten Substituenten gegebenenfalls substituiertes Phenyl oder Naphthyl, so kommen hierfür die Reste wie, Phenyl, Benzyl, Cumyl, $\alpha$- oder $\beta$-Naphthyl in Frage. Neben $C_1$-$C_4$-Alkylresten können die Phenyl oder Naphthylreste auch durch Halogen, insbesondere Cl oder Br, sowie $C_1$-$C_4$-Alkoxy substituiert sein.

Bedeuten Substituenten Halogen, so kommen hierfür F, Br und besonders bevorzugt Cl in Frage.

Verbindungen, welche einen Rest der Formel IV enthalten, können eine lineare und auch ringförmige Struktur haben. Die einzelnen Monomereneinheiten des Restes der Formel IV sind statistisch verteilt.

Bevorzugt sind Verbindungen der Formel (I), worin mindestens einer der Reste $R_2$, $R_3$ und $R_5$ von H verschieden ist.

Bevorzugt sind weiterhin Verbindungen der Formel (I), worin der Rest $R_3$ in para-Stellung zu dem CO-Rest angeordnet ist und/oder der Rest $R_4$ in ortho-Stellung zu dem CO-Rest angeordnet ist.

Bevorzugt sind weiterhin Verbindungen der Formel (I), worin i eins oder zwei, besonders bevorzugt eins, ist.

Bevorzugt sind weiterhin Verbindungen der Formel (I), worin L, wenn i eins ist, H, $C_1$-$C_{20}$-Alkyl, mit ein oder zwei -OH, mit $C_1$-$C_{12}$-Alkoxy, mit Phenoxy, mit $C_2$-$C_{20}$-Alkylcarbonyl, oder mit Phenyl substituiertes $C_2$-$C_{20}$-Alkyl, Glycidyl, mit ein bis sechs O-Atomen unterbrochenes $C_4$-$C_{20}$-Alkyl, $C_2$-$C_{18}$-Alkenyl, gegebenenfalls substituiertes Phenyl oder Naphthyl oder eine Gruppe der Formel II, III oder IV

$-(CH_2)_j-CH(OH)-(CH_2)_k-O-R_9$ (II) mit j, k =0-4;
$-(CH_2)_m-COOR_9$ (III) mit m = 1-4;

wobei $R_9$ $C_1$-$C_{18}$-Alkyl oder Phenyl ist;

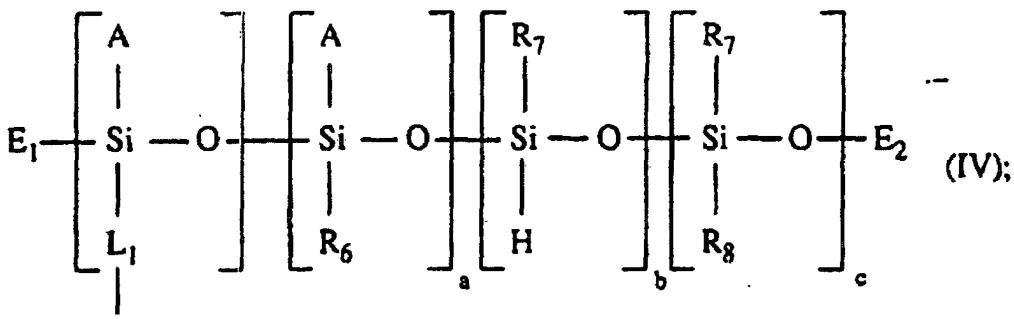

wobei a = 0-50, b = 0-50 und c = 0-50 ist;
A $R_7$ oder-O-Si$(R_7)_3$;
$E_1$ OH, $C_1$-$C_{12}$-Alkyl, $C_1$-$C_4$-Alkoxy, Cyclohexyl, Phenyl oder -O-Si$(R_7)_3$;
$E_2$ H, $C_1$-$C_{12}$-Alkyl, Cyclohexyl, Phenyl, -Si$(R_7)_3$ oder -Si$(R_6)(R_7)_2$;
wobei $E_1$ und $E_2$ zusammen auch eine direkte Bindung bedeuten können;
$L_1$ eine direkte Bindung oder eine zweiwertige Gruppe der Formeln $-C_nH_{2n}-$, $-(CH_2)_n-O-$, $-CH_2CH(OH)CH_2-O-$ oder $-CH_2CH(OH)CH_2-O-(CH_2)_3-$, mit n = 1-4;
$R_6$ ein Rest der Formel

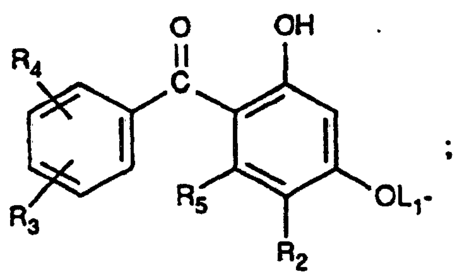

$R_7$ $C_1$-$C_{12}$-Alkyl, $C_1$-$C_4$-Alkoxy, Cyclohexyl oder Phenyl; und
$R_8$ $C_1$-$C_{18}$-Alkyl, $C_1$-$C_4$-Alkoxy, $C_5$-$C_7$-Cycloalkyl oder Phenyl ist.

Bevorzugt sind weiterhin Verbindungen der Formel (I), worin $R_3$-$SR_{10}$ oder -$SO_2R_{10}$ ist.

Bevorzugt sind weiterhin Verbindungen der Formel (I), worin

$R_{10}$ $C_1$-$C_{20}$-Alkyl, mit -OH oder mit $C_1$-$C_{12}$-Alkoxy substituiertes $C_2$-$C_{20}$-Alkyl, $C_3$-$C_{18}$-Alkenyl, $C_5$-$C_{12}$-Cycloalkyl, $C_7$-$C_{15}$-Phenylalkyl, $C_6$-$C_{10}$-Aryl oder mit ein oder zwei $C_1$-$C_4$-Alkyl-Gruppen substituiertes $C_6$-$C_{10}$-Aryl.

Besonders bevorzugt sind Verbindungen der Formel (I), worin

i eins ist;
L H, $C_1$-$C_{12}$-Alkyl, $C_2$-$C_{12}$-Alkenyl, Phenyl, Naphthyl oder ein Rest der Formel II, III oder IV
$-(CH_2)_j-CH(OH)-(CH_2)_k-O-R_9$ (II) mit j, k = 0-4;
$-(CH_2)_m-COOR_9$ (III) mit m = 1-4;

wobei $R_9$ $C_1$-$C_{18}$-Alkyl oder Phenyl ist;

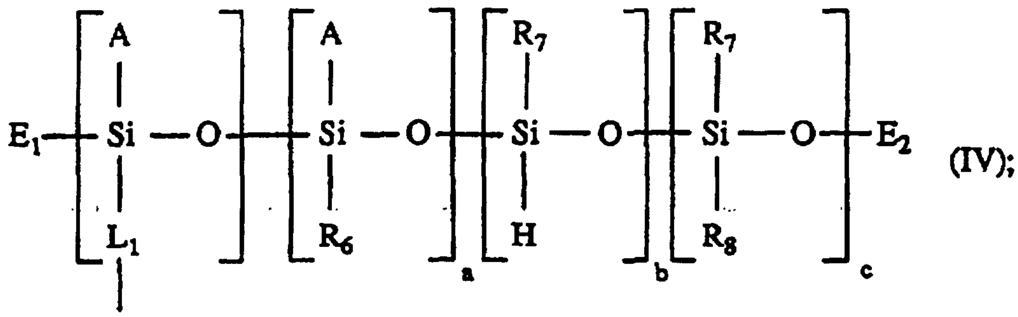

wobei a = 0-50, b = 0 und c = 0-50 ist;
A $R_7$ oder -O-Si$(R_7)_3$;
$E_1$ OH, $C_1$-$C_{12}$-Alkyl, $C_1$-$C_4$-Alkoxy, Cyclohexyl, Phenyl oder -O-Si$(R_7)_3$;
$E_2$ H, $C_1$-$C_{12}$-Alkyl, Cyclohexyl, Phenyl, -Si$R_6(R_7)_2$ oder -Si$(R_7)_3$;
wobei $E_1$ und $E_2$ zusammen auch eine direkte Bindung bedeuten können;
$L_1$ eine direkte Bindung oder eine zweiwertige Gruppe der Formeln $-C_nH_{2n}-$, $-(CH_2)_n-O-$ oder $-CH_2CH(OH)CH_2-O-(CH_2)_3-$, mit n = 1-4;
$R_6$ ein Rest der Formel

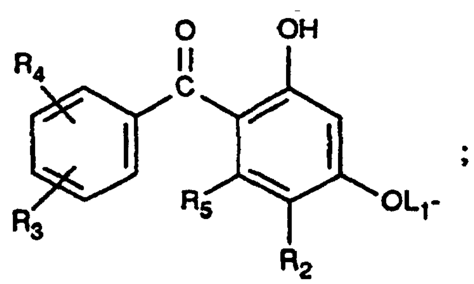

$R_7$ $C_1$-$C_{12}$-Alkyl, $C_1$-$C_4$-Alkoxy, Cyclohexyl oder Phenyl; und
$R_8$ $C_1$-$C_{18}$-Alkyl, $C_1$-$C_4$-Alkoxy, $C_5$-$C_7$-Cycloalkyl oder Phenyl ist;

$R_2$ $C_1$-$C_{12}$-Alkyl oder Cl oder, wenn $R_5$ -OL oder Cl oder $R_3$ $-SR_{10}$ oder $-SO_2R_{10}$ ist, auch H;
$R_3$ $-SR_{10}$, $-SO_2R_{10}$
$R_4$ H, $C_1$-$C_{12}$-Alkyl oder $C_2$-$C_{12}$-Alkenyl;
$R_5$ H, -OL oder Cl; und
$R_{10}$ $C_1$-$C_{20}$-Alkyl, mit -OH oder mit $C_1$-$C_{12}$-Alkoxy substituiertes $C_2$-$C_{20}$-Alkyl, $C_3$-$C_{18}$-Alkenyl, $C_5$-$C_{12}$-Cycloalkyl, $C_7$-$C_{15}$-Phenylalkyl, $C_6$-$C_{10}$-Aryl oder mit ein oder zwei $C_1$-$C_4$-Alkyl-Gruppen substuiertes $C_6$-$C_{10}$-Aryl

bedeutet.

Ganz besonders bevorzugt sind Verbindungen der Formel (I) worin

E) i eins ist;

L $C_1$-$C_{12}$-Alkyl;
$R_2$, $R_4$ und $R_5$ H;
$R_3$ $-SR_{10}$ oder $-SO_2R_{10}$; und
$R_{10}$ $C_1$-$C_{12}$-Alkyl oder Phenyl bedeutet.

In bestimmten Fällen kann es von Vorteil sein, zwei oder mehrere oben genannter Benzophenone zu verwenden. Üblicherweise ist das Gewichtsverhältnis der Benzophenone in der Mischung von 5:1 bis 1:5, wobei Verhältnisse von 3:1 bis 1:3 bevorzugt sind.

In dem erfindungsgemässen Verfahren können die Anstrichzusammensetzungen die nachfolgend näher beschriebenen Bestandteile enthalten. Ein Gegenstand der Erfindung sind auch Schutzanstrichzusammensetzungen für Holz enthaltend eine Verbindung der Formel (I). Eine mögliche Einteilung der Schutzanstriche für Holz ist die Unterscheidung zwischen Grund- oder Imprägnieranstrich und Deckanstrich wie im folgenden erläutert. Bevorzugt ist eine

Deckanstrichzusammensetzung und das Verfahren, welches eine Deckanstrichzusammensetzung verwendet, worin eine Verbindung der Formel I enthalten ist. Die weiter oben angegebenen Bevorzugungen für die Verbindungen der Formel I im erfindungsgemässen Verfahren gelten gleichermassen auch für die Schutzanstrichzusammensetzungen.

Wird ein Grundanstrich oder Imprägnieranstrich aufgebaut, so soll dieser in die Holzoberfläche eindringen, d.h. von relativ niedriger Viskosität sein. Beispiele für hierfür brauchbare Lösungsmittel sind aliphatische Kohlenwasserstoffe wie z.B. bestimmte Fraktionen von Benzin. Weitere Beispiele für Lösungsmittel sind aromatische Kohlenwasserstoffe, wie z.B. Toluol oder Xylol; Alkohole, wie z.B. Methanol, Ethanol, Isopropanol oder Butanol; Ester, wie z.B. Ethylacetat oder Butylacetat; oder Ketone, wie z.B. Aceton, Methylethylketon oder Methyl-isobutyl-keton. Diese Lösungsmittel verdampfen bei Raumtemperatur und bleiben daher nicht im Holz. Man kann aber auch hochsiedende Flüssigkeiten zusetzen, die im Holz bleiben, wie z.B. höhere Alkanole, Glykole, Glykolether, Glykolester oder Polyglykole. Der Grundanstrich kann auch ein Bindemittel enthalten, wie sie für Holzanstriche üblich sind. Das können z.B. Alkydharze und modifizierte Alkydharze sein, selbstvernetzende oder fremdverntzende Acrylatharze, Polyesterharze, trocknende Oele, Phenolharze, Nitrocellulose oder Mischungen davon.

Der Grundanstrich kann auch einen oder mehrere Phosphorigsäureester enthalten. Beispiele für solche Phosphorigsäureester sind:

> Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Bis-(2,4-di-tert.butylphenyl)pentaerythritdiphosphit, Tristearyl-sorbit-triphosphit. Besonders bevorzugt ist das Tris-(2,4-di-tert.butylphenyl)-phosphit.

Der Grundanstrich kann auch Konservierungsmittel, z.B. Fungizide oder Insektizide enthalten. Beispiele für verwendbare Fungizide sind Tributylzinnoxid, Phenylquecksilbersalze, Kupfernaphthenat, 1-Chlornaphthalin oder Pentachlorphenol. Beispiele für verwendbare Insektizide sind DDT, Dieldrin, Lindan, Parathion oder Phoxim.

Weitere Zusätze, die im Grundanstrich enthalten sein können, sind Härtungsbeschleuniger (Trockner) für die Bindemittel, Farbstoffe oder auch Pigmente in geringer Menge.

Der Grundanstrich kann auf das Holz nach den hierfür üblichen Methoden aufgebracht werden, beispielsweise durch Tauchen, Streichen oder Besprühen.

Der Grundanstrich kann auch ein wässriger Anstrich sein. Anstelle des organischen Lösungsmittels treten dann als Vehikel Wasser oder Mischungen von Wasser und einem wasserlöslichen organischen Lösungsmittel. Die Inhaltsstoffe können in diesem Vehikel gelöst oder dispergiert sein.

Als Deckanstrich kann jeder für die Lackierung von Holz geeignete Lack verwendet werden. Er enthält meist ein Bindemittel, gelöst oder dispergiert in einem organischen Lösungsmittel oder in Wasser oder in einem Wasser/Lösungsmittel-Gemisch. Das Bindemittel kann z.B. ein lufttrocknendes bzw. bei Raumtemperatur härtbares Lackharz sein. Beispiele hierfür sind Nitrocellulose, Polyvinylacetat, Polyvinylchlorid, ungesättigte Polyesterharze, Polyacrylate, Polyurethane, Epoxidharze, Phenolharze, insbesondere aber Alkydharze. Das Bindemittel kann auch ein Gemisch verschiedener Lackharze sein. Soweit es sich um härtbare Bindemittel handelt, werden sie meist zusammen mit einem Härter und/oder einem Härtungsbeschleuniger eingesetzt. Dabei kann es sich um 1-Komponenten- oder Mehrkomponenten-Systeme handeln.

Als organische Lösungsmittel können die für Lacke üblichen Lösungsmittel verwendet werden, z.B. aliphatische, aromatische oder cycloaliphatische Kohlenwasserstoffe, Alkohole, Ester, Ketone oder Chlorkohlenwasserstoffe.

Wasser/Lösungsmittel-Gemische sind z.B. Gemische von Wasser mit niedrigen Alkoholen, Glykolen oder Glykolethern.

Der Deckanstrich kann auch eine durch aktinische Strahlung härtbare Zusammensetzung von photopolymerisierbaren Verbindungen sein. Beispiele hierfür sind Gemische von Acrylaten bzw. Methacrylaten, ungesättigte Polyester/Styrol-Gemische oder Gemische sonstiger ethylenisch ungesättigter Monomeren bzw. Oligomeren.

Der Deckanstrich kann einen löslichen Farbstoff und/oder ein Pigment und/oder einen Füllstoff enthalten. Das Pigment kann ein organisches, anorganisches oder metallisches Pigment sein. Der Füllstoff kann z.B. Talk, Kaolin, Calciumcarbonat oder Aluminiumsilicat sein. Vorzugsweise ist der Deckanstrich ein Klarlack, das heisst, er enthält keine ungelösten Bestandteile.

Der Deckanstrich besteht in der Hauptmenge aus einem Lösungsmittel und enthält z.B. 0,1 bis 30 Gew.-%, vorzugsweise 0,1 bis 5 Gew.-% des Benzophenons der Formel (I), jeweils bezogen auf den Feststoffgehalt des Anstrichs.

Neben dem Benzophenon der Formel (I) können dem Holzschutzanstrich noch weitere Lichtschutzmittel, insbesondere sterisch gehinderte Amine vom Typ der 2,2,6,6-Tetraalkylpiperidine, zugegeben werden wie beispielsweise:

> 2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tertamyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'tert-Butyl-2'-hydroxy-5'-(2-isooctyloxycarbonylethyl)phenyl-benzotriazol, 2,2'-Methylenbis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; $[R-CH_2CH_2-COO(CH_2)_3]_2$ mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3'5-Di-tert-butyl-4-hydroxybenzoesäure-2,4-di-tertbutylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäure-methylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'-di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tertbutyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

Bevorzugt sind dabei Lichtschutzmittel aus den Klassen 2.1, 2.2, 2.6 und 2.8.

Besonders wirkungsvoll ist der Zusatz eines Gemisches eines UV-Absorbers, wie z.B. einer Verbindung der oben angegebenen Klassen 2.1, 2.2, 2.3, 2.4, 2.7 und 2.8, bevorzugt 2.1 und 2.8, mit einem sterisch gehinderten Amin, wie z.B. einer Verbindung der oben angegebenen Klasse 2.6.

Auch können dem Holzschutzanstrich Antioxidantien zugegeben werden, wie beispielsweise:

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Ditert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Dioctylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tertbutyl-4-hydroxyanisol, 3,5-Di-tert-butyl-4-hydroxyphenyl-stearat, Bis-(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

1.6. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylenbis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tent-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien,Bis-[2-(3'-tert-butyl-2'-hydroxy-5'-methyl-benzyl)-6-tert-butyl-4-methyl-phenyl]-terephthalat,1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-ditert-butyl-4-hydroxybenzyl)-amin, Bis-(4-tert-butyl-3-hydroxy-2,6-dimethylbenzyl)-dithioterephthalat, Bis-(3,5-di-tert-butyl-4-hydroxybenzyl)-sulfid, Isooctyl-3,5-di-tertbutyl-4-hydroxybenzyl-mercaptoacetat.

1.8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Didodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

1.9. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-ditertbutyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein- oder mehrwertigen Alkoholen, wie z. B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N, N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-ditert-butyl-4-hydroxyphenylpropionyl)-trimethylendiamin, N,N'-Bis-(3,5-di-tert-butyl-4-hydroxyphenylpropionyl)-hydrazin.

Der Deckanstrich kann weiter in der Lacktechnologie übliche Zusätze enthalten, wie z.B. Verlaufhilfsmittel, Thioxotropiemittel, Flammschutzmittel, Antioxidantien oder lösliche Farbstoffe.

Die Applikation der Holzschutzanstriche erfolgt nach den üblichen Methoden der Holzlackierung, sie kann z. B. durch Spritzen, Streichen, Bürsten, Giessen oder Tauchen geschehen.

Der Deckanstrich kann in mehreren Anstrichen aufgetragen werden, damit er eine genügende Schichtdicke erhält. Es hängt von dem beabsichtigen Verwendungszeck ab, wie dick der Deckanstrich sein soll.

Ein weiterer Gegenstand der vorliegenden Erfindung ist auch ein Benzophenon der Formel I

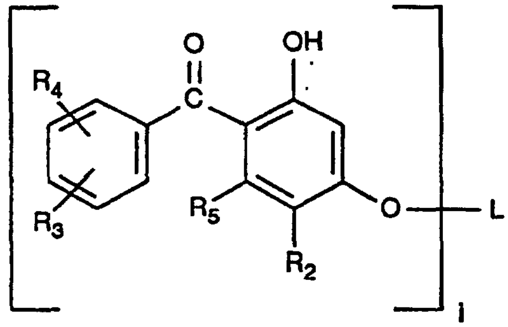 (I),

worin

i eine Zahl von eins bis vier;

L, wenn i eins ist, H, $C_1$-$C_{20}$-Alkyl, mit ein oder zwei -OH, mit Carboxyl, mit $C_1$-$C_{12}$-Alkoxy, mit Phenoxy, mit $C_2$-$C_{20}$-Alkylcarbonyl, mit $C_3$-$C_{20}$-Alkenylcarbonyl, mit $C_2$-$C_{20}$-Alkanoyloxy, mit $C_3$-$C_{20}$-Alkenoyloxy oder mit Phenyl substituiertes $C_2$-$C_{20}$-Alkyl, Glycidyl, mit ein bis sechs O-Atomen unterbrochenes oder mit ein oder zwei Carbonyloxy- oder Oxycarbonyl-Gruppen unterbrochenes $C_4$-$C_{20}$-Alkyl, $C_2$-$C_{18}$-Alkenyl, $C_2$-$C_{18}$-Alkanoyl, $C_3$-$C_{18}$-Alkenoyl, Benzoyl, mit ein oder zwei $C_1$-$C_4$-Alkyl-Gruppen substituiertes Benzoyl, gegebenenfalls substituiertes Phenyl oder Naphthyl oder eine Gruppe der Formel II, III, IV, V oder VI

$-(CH_2)_j-CH(OH)-(CH_2)_k-O-R_9$ (II) mit j, k = 0-4;

$-(CH_2)_m-COOR_9$ (III) mit m = 1-4;

wobei $R_9$ $C_1$-$C_{18}$-Alkyl oder Phenyl ist;

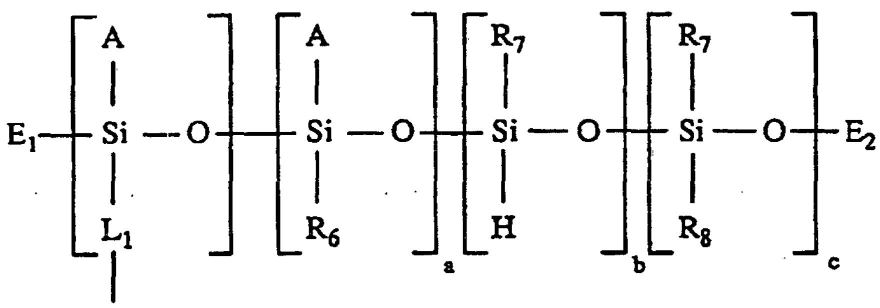 (IV);

wobei a = 0-50, b = 0-50 und c = 0-50 ist;

A $R_7$ oder $-O-Si(R_7)_3$;

$E_1$ OH, $C_1$-$C_{12}$-Alkyl, $C_1$-$C_4$-Alkoxy, Cyclohexyl, Phenyl oder $-O-Si(R_7)_3$;

$E_2$ H, $C_1$-$C_{12}$-Alkyl, Cyclohexyl, Phenyl, $-Si(R_7)_3$ oder $-Si(R_6)(R_7)_2$;

wobei $E_1$ und $E_2$ zusammen auch eine direkte Bindung bedeuten können;

$L_1$ eine direkte Bindung oder eine zweiwertige Gruppe der Formeln $-C_nH_{2n}-$, $-(CH_2)_n-O-$, $-CH_2CH(OH)CH_2-O-$ oder $-CH_2CH(OH)CH_2-O-(CH_2)_3-$, mit n = 1-4;

$R_6$ ein Rest der Formel

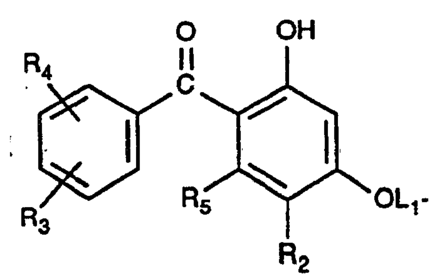 ;

$R_7$ $C_1$-$C_{12}$-Alkyl, $C_1$-$C_4$-Alkoxy, Cyclohexyl oder Phenyl; und
$R_8$ $C_1$-$C_{18}$-Alkyl, $C_1$-$C_4$-Alkoxy, $C_5$-$C_7$-Cycloalkyl oder Phenyl ist;

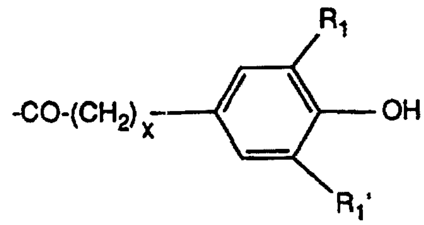
(V)

wobei x 0, 1 oder 2 ist;

$R_1$ $C_1$-$C_{12}$-Alkyl oder $C_5$-$C_8$-Cycloalkyl; und
$R_1$' sekundäres oder tertiäres $C_3$-$C_{12}$-Alkyl oder $C_5$-$C_8$-Cycloalkyl ist;

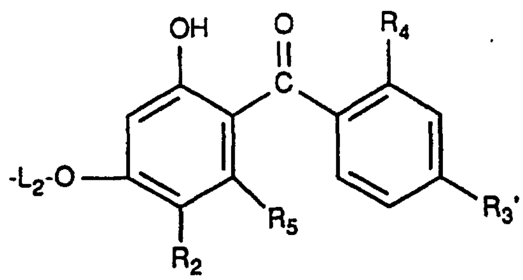
(VI)

wobei $L_2$ die Bedeutung von L, wenn i zwei ist, hat; und
$R_3$' H oder $C_1$-$C_{18}$-Alkyl ist;

L, wenn i zwei ist, $C_1$-$C_{12}$-Alkylen, mit -OH, mit $C_1$-$C_8$-Alkoxy, mit $C_2$-$C_{20}$-Alkoxycarbonyl, mit $C_2$-$C_{20}$-Alkanoyloxy, mit $C_3$-$C_{20}$-Alkenoyloxy oder mit einer Silyl-Gruppe der Formel (IV) substituiertes $C_3$-$C_{12}$-Alkylen, mit ein bis sechs O-Atomen unterbrochenes oder mit ein oder zwei Carbonyloxy- oder Oxycarbonyl-Gruppen unterbrochenes $C_4$-$C_{20}$-Alkylen, o-Xylylen, m-Xylylen, p-Xylylen, iso-Phthaloyl, Phthaloyl, Terephthaloyl oder $\alpha,\omega$-$C_4$-$C_{12}$-Alkandioyl;
L, wenn i drei ist, $C_3$-$C_{12}$-Alkantriyl, $C_3$-$C_{12}$-Alkantrioyl, Trimellitoyl oder mit drei Carbonyloxy- oder Oxycarbonyl-Gruppen unterbrochenes $C_6$-$C_{20}$-Alkantriyl;
L, wenn i vier ist, $C_4$-$C_{16}$-Alkantetrayl, $C_4$-$C_{16}$-Alkantetroyl, Pyromellitoyl oder mit vier Carbonyloxy- oder Oxycarbonyl-Gruppen unterbrochenes $C_8$-$C_{24}$-Alkantetrayl;

$R_2$ H, $C_1$-$C_{18}$-Alkyl oder Cl;
$R_3$ -$SOR_{10}$ oder -$SO_2R_{10}$;
$R_4$ H, -OH, $C_1$-$C_{18}$-Alkyl oder $C_2$-$C_{18}$-Alkenyl;
$R_5$ H, -OL, mit L in der Bedeutung wenn i eins ist, oder Cl; und
$R_{10}$ $C_1$-$C_{20}$-Alkyl, mit -OH, mit $C_1$-$C_{12}$-Alkoxy, mit einer Silyl-Gruppe der Formel (IV), mit $C_2$-$C_{12}$-Alkanoyloxy, mit $C_3$-$C_{12}$-Alkenoyloxy oder mit Halogen substituiertes $C_2$-$C_{20}$-Alkyl, $C_3$-$C_{18}$-Alkenyl, $C_5$-$C_{12}$-Cycloalkyl, $C_7$-$C_{15}$-Phenylalkyl, $C_6$-$C_{10}$-Aryl, mit ein oder zwei $C_1$-$C_4$-Alkyl-Gruppen substituiertes $C_6$-$C_{10}$-Aryl oder 1,1,2,2-Tetrahydroperfluoro-$C_6$-$C_{16}$-alkyl;

bedeutet.

Die erfindungsgemässen Verbindungen, für die die weiter oben genannten Bevorzugungen analog gelten, lassen sich durch in der organischen Chemie übliche Methoden aus den schon aus dem Stand der Technik bekannten Verbindungen herstellen.

Die folgenden Beispiele zeigen mögliche Ausführungen der Erfindung im Detail. Darin bedeuten Teile Gewichtsteile und % Gewichts-%.

Beispiel 1:

**2-Hydroxy-4'-phenylthio-4-dodecyloxybenzophenon**

Eine Mischung von 77,1 g (0,31 mol) 2,4-Dihydroxy-4'-chlorobenzophenon, 44,4 g (0,4 mol) Thiophenol, 51,4 g (0,37 mol) Kaliumcarbonat in 600 ml 2-Methyl-2-pyrrolidon wird unter Rühren erwärmt und 7 h bei 150°C gehalten. Nach Abkühlung wird das Reaktionsgemisch mit 125 ml HCl 10 % behandelt und mit Essigsäure-ethylester extrahiert. Die erhaltene Lösung wird mit Wasser und anschliessend mit einer gesättigten Natriumchloridlösung gewaschen. Der nach Trocknen mit $MgSO_4$, Abfiltrieren und Eindampfen verbleibende Rückstand wird aus $CH_2Cl_2$/Hexan umkristallisiert. Man erhält 52,6 g (52,6 %) von 2,4-Dihydroxy-4'-phenylthiobenzophenon als beige Kristalle mit dem Schmelzpunkt 114-116°C.

Eine Mischung von 7,7 g (24 mmol) 2,4-Dihydroxy-4'-phenylthiobenzophenon, 6,6 g (26,4 mmol) 1-Bromdodecan, 3,3 g (24 mmol) Kaliumcarbonat, 40 mg (0,024 mmol) Kaliumjodid in 100 ml Xylol und 50 ml N,N-Dimethylacetamid wird 3 h bei 130°C gerührt. Nach Abkühlung wird das Reaktionsgemisch mit 100 ml Wasser behandelt und mit Essigsäure-ethylester extrahiert. Die erhaltene Lösung wird mit Wasser und anschliessend mit einer gesättigten Natriumchloridlösung gewaschen. Der nach Trocknen mit $MgSO_4$, Abfiltrieren und Eindampfen verbleibende Rückstand wird aus Isopropanol umkristallisiert. Man erhält 10,5 g (89 %) der Verbindung als leicht gelbe Kristalle mit dem Schmelzpunkt 59-60°C.

| | | | | |
|---|---|---|---|---|
| Analyse | Ber. | C 75,88 | H 7,81 | S 6,53 |
| ($C_{31}H_{38}O_3S$) | Gef. | C 75.90 | H 7.81 | S 6,68 |

Beispiel 2:

Die erfindungsgemässen Verbindungen (%-Angaben in der Tabelle 1 bezüglich Festkörper des Klarlackes) werden in ca. 5 g Xylol vorgelöst und in einen Klarlack folgender Zusammensetzung eingearbeitet:

| | |
|---|---|
| Jägalyd Antihydro® (60 % in Testbenzin)[1)] | 53,48 |
| Jägalyd Antihydro Thix® (50 % in Testbenzin/Solv.)[2)] | 10,69 |
| Jägalyd Antihydro Trockner®[3)] | 1,92 |
| Testbenzin | 33,27 |
| Ascinin P® [4)] | 0,32 |
| Luactim M® [5)] | 0,32 |
| | 100,0 |

[1)]Alkydharz der Firma Ernst Jäger GmbH & Co; DE
[2)]Thixotropiemittel der Firma Ernst Jäger GmbH & Co; DE
[3)]Härtungskatalysator der Firma Ernst Jäger GmbH & Co; DE
[4)]Hautverhinderungsmittel der Firma Bayer AG; DE
[5)]Hautverhinderungsmittel der Firma BASF AG; DE

Der Klarlack wird auf ein mit einer Imprägnierlasur (siehe unten) beschichtetes Holzsubstrat (Fichte) aufgebracht (3 mal mit Pinsel, zwischen jedem Auftrag 24 h Trocknung bei Raumtemperatur). Nach 2 Wochen Lagerung bei Raumtemperatur werden die Proben in einem Xenon-Weatherometer® (Fa. Atlas Corp.) bewittert (Zyklus 7; 102: 18 [Verhältnis Trocken- zu Feuchtphase in Minuten]). Die Bestimmung der 60° Glanzes erfolgt nach DIN 67530; der Yellowness-Index (YI) wird nach ASTM D 1925-88 bestimmt.

**Imprägnierlasur**

| | |
|---|---|
| Jägalyd Antihydro® (60 % in Testbenzin)[1)] | 27,00 |
| Jägalyd Anohydro Trockner® [3)] | 1,00 |
| Ethylglykol | 3,00 |

[1)]Alkydharz der Firma Ernst Jäger GmbH & Co; DE
[3)]Härtungskatalysator der Firma Ernst Jäger GmbH & Co; DE

(fortgesetzt)

| | |
|---|---|
| Traetex 293® [6] | 1,30 |
| Luactim M® [5] | 0,20 |
| Testbenzin | 52,50 |
| Xylol | 15,00 |
| | 100,0 |

[5] Hautverhinderungsmittel der Firma BASF AG; DE

[6] Fungizid der Firma Acima AG; CH

Auftrag der Imprägnierlasur: 1 mal mit Pinsel, dann 24 h Lagerung bei Raumtemperatur bevor der Klarlack appliziert wird.

Tabelle 1:

| Zunahme von YI nach 801 Stunden Bewitterung | |
|---|---|
| a) unstabilisiert | 26,7 |
| e) 2% der Verbindung von Bsp. 1 | 5,6 |

Die erfindungsgemäss stabilisierten Proben zeigen eine bessere Rissfreiheit (höhere Glanz-Werte) und eine geringere Vergilbung (kleinere Zunahme des YI-Wertes) als die Vergleichsproben.

## Patentansprüche

1. Verfahren zum Schützen einer Holzoberfläche gegen Schädigung durch Licht durch Behandlung mit einer Anstrichzusammensetzung, enthaltend ein Benzophenon der Formel I

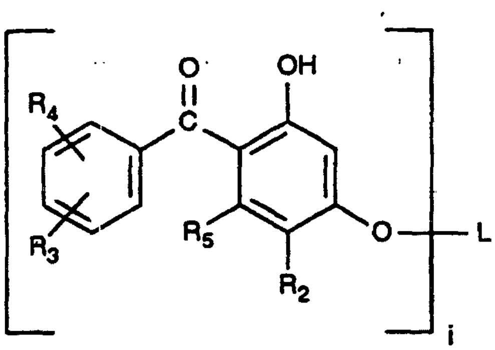

(I),

worin

i eine Zahl von eins bis vier,

L, wenn i eins ist, H, $C_1$-$C_{20}$-Alkyl, mit ein oder zwei -OH, mit Carboxyl, mit $C_1$-$C_{12}$-Alkoxy, mit Phenoxy, mit $C_2$-$C_{20}$-Alkylcarbonyl, mit $C_3$-$C_{20}$-Alkenylcarbonyl, mit $C_2$-$C_{20}$-Alkanoyloxy, mit $C_3$-$C_{20}$-Alkenoyloxy oder mit Phenyl substituiertes $C_2$-$C_{20}$-Alkyl, Glycidyl, mit ein bis sechs O-Atomen unterbrochenes oder mit ein oder zwei Carbonyloxy- oder Oxycarbonyl-Gruppen unterbrochenes $C_4$-$C_{20}$-Alkyl, $C_2$-$C_{18}$-Alkenyl, $C_2$-$C_{18}$-Alkanoyl, $C_3$-$C_{18}$-Alkenoyl, Benzoyl, mit ein oder zwei $C_1$-$C_4$-Alkyl-Gruppen substituiertes Benzoyl, gegebenenfalls substituiertes Phenyl oder Naphthyl oder eine Gruppe der Formel II, III, IV, V oder VI

$-(CH_2)_j-CH(OH)-(CH_2)_k-O-R_9$ (II) mit j, k =0-4;

$-(CH_2)_m-COOR_9$ (III) mit m = 1-4;

wobei $R_9$ $C_1$-$C_{18}$-Alkyl oder Phenyl ist;

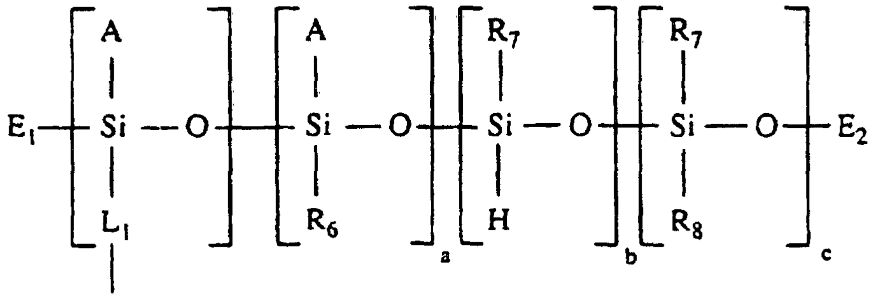 (IV);

wobei a = 0-50, b = 0-50 und c = 0-50 ist;
A $R_7$ oder $-O-Si(R_7)_3$;
$E_1$ OH, $C_1$-$C_{12}$-Alkyl, $C_1$-$C_4$-Alkoxy, Cyclohexyl, Phenyl oder $-O-Si(R_7)_3$;
$E_2$ H, $C_1$-$C_{12}$-Alkyl, Cyclohexyl, Phenyl, $-Si(R_7)_3$ oder $-Si(R_6)(R_7)_2$;
wobei $E_1$ und $E_2$ zusammen auch eine direkte Bindung bedeuten können;
$L_1$ eine direkte Bindung oder eine zweiwertige Gruppe der Formeln $-C_nH_{2n}-$, $-(CH_2)_n-O-$, $-CH_2CH(OH)CH_2-O-$ oder $-CH_2CH(OH)CH_2-O-(CH_2)_3-$, mit n = 1-4;
$R_6$ ein Rest der Formel

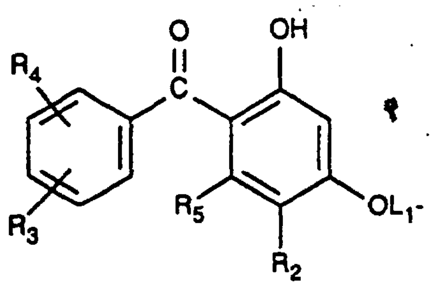 ;

$R_7$ $C_1$-$C_{12}$-Alkyl, $C_1$-$C_4$-Alkoxy, Cyclohexyl oder Phenyl; und
$R_8$ $C_1$-$C_{18}$-Alkyl, $C_1$-$C_4$-Alkoxy, $C_5$-$C_7$-Cycloalkyl oder Phenyl ist;

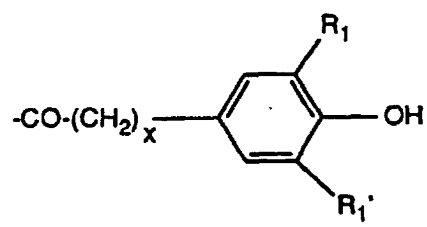 (V)

wobei x 0, 1 oder 2 ist;
$R_1$ $C_1$-$C_{12}$-Alkyl oder $C_5$-$C_8$-Cycloalkyl; und
$R_1'$ sekundäres oder tertiäres $C_3$-$C_{12}$-Alkyl oder $C_5$-$C_8$-Cycloalkyl ist;

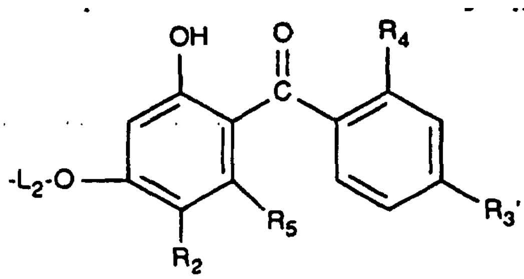 (VI)

wobei $L_2$ die Bedeutung von L, wenn i zwei ist, hat; und
$R_3$' H oder $C_1$-$C_{18}$-Alkyl ist;

L, wenn i zwei ist, $C_1$-$C_{12}$-Alkylen, mit -OH, mit $C_1$-$C_8$-Alkoxy, mit $C_2$-$C_{20}$-Alkoxycarbonyl, mit $C_2$-$C_{20}$-Alkanoyloxy, mit $C_3$-$C_{20}$-Alkenoyloxy oder mit einer Silyl-Gruppe der Formel (IV) substituiertes $C_3$-$C_{12}$-Alkylen, mit ein bis sechs O-Atomen unterbrochenes oder mit ein oder zwei Carbonyloxy- oder Oxycarbonyl-Gruppen unterbrochenes $C_4$-$C_{20}$-Alkylen, o-Xylylen, m-Xylylen, p-Xylylen, iso-Phthaloyl, Phthaloyl, Terephthaloyl oder $\alpha,\omega$-$C_4$-$C_{12}$-Alkandioyl;
L, wenn i drei ist, $C_3$-$C_{12}$-Alkantriyl, $C_3$-$C_{12}$-Alkantrioyl, Trimellitoyl oder mit drei Carbonyloxy- oder Oxycarbonyl-Gruppen unterbrochenes $C_6$-$C_{20}$-Alkantriyl;
L, wenn i vier ist, $C_4$-$C_{16}$-Alkantetrayl, $C_4$-$C_{16}$-Alkantetroyl, Pyromellitoyl oder mit vier Carbonyloxy- oder Oxycarbonyl-Gruppen unterbrochenes $C_8$-$C_{24}$-Alkantetrayl;
$R_2$ H, $C_1$-$C_{18}$-Alkyl oder Cl;
$R_3$ -$SR_{10}$, -$SOR_{10}$ oder -$SO_2R_{10}$;
$R_4$ H, -OH, $C_1$-$C_{18}$-Alkyl oder $C_2$-$C_{18}$-Alkenyl;
$R_5$ H, -OL, mit L in der Bedeutung wenn i eins ist, oder Cl; und
$R_{10}$ $C_1$-$C_{20}$-Alkyl, mit -OH, mit $C_1$-$C_{12}$-Alkoxy, mit einer Silyl-Gruppe der Formel (IV), mit $C_2$-$C_{12}$-Alkanoyloxy, mit $C_3$-$C_{12}$-Alkenoyloxy oder mit Halogen substituiertes $C_2$-$C_{20}$-Alkyl, $C_3$-$C_{18}$-Alkenyl, $C_5$-$C_{12}$-Cycloalkyl, $C_7$-$C_{15}$-Phenylalkyl, $C_6$-$C_{10}$-Aryl, mit ein oder zwei $C_1$-$C_4$-Alkyl-Gruppen substituiertes $C_6$-$C_{10}$-Aryl oder 1,1,2,2-Tetrahydroperfluoro-$C_6$-$C_{16}$-alkyl;

bedeutet.

**2.** Verfahren gemäss Anspruch 1, **dadurch gekennzeichnet, dass** das Benzophenon der Formel I entspricht, worin

L $C_1$-$C_{12}$-Alkyl;
$R_2$, $R_4$ und $R_5$ H;
$R_3$ -$SR_{10}$ oder -$SO_2R_{10}$; und
$R_{10}$ $C_1$-$C_{12}$-Alkyl oder Phenyl bedeutet.

**3.** Verfahren gemäss Anspruch 1, **dadurch gekennzeichnet, dass** es sich bei der eine Verbindung der Formel (I) enthaltenden Anstrichzusammensetzung um einen Decklack handelt.

**4.** Verfahren gemäss Anspruch 1, **dadurch gekennzeichnet, dass** es sich bei der eine Verbindung der Formel (I) enthaltenden Anstrichzusammensetzung um einen Decklack handelt, welcher ein Klarlack ist.

**5.** Verfahren gemäss Anspruch 1, **dadurch gekennzeichnet, dass** es sich bei der eine Verbindung der Formel (I) enthaltenden Anstrichzusammensetzung um einen Decklack handelt, der ein Bindemittel auf Basis eines Alkydharzes enthält.

**6.** Verfahren gemäss Anspruch 1, **dadurch gekennzeichnet, dass** es sich bei der eine Verbindung der Formel (I) enthaltenden Anstrichzusammensetzung um einen Decklack handelt, der ein oder mehrere weitere Lichtschutzmittel enthält.

**7.** Verfahren gemäss Anspruch 1, **dadurch gekennzeichnet, dass** es sich bei der eine Verbindung der Formel (I) enthaltenden Anstrichzusammensetzung um einen Decklack handelt, der als zusätzliches Lichtschutzmittel ein sterisch gehindertes Amin enthält.

**8.** Verfahren gemäss Anspruch 1, **dadurch gekennzeichnet, dass** es sich bei der eine Verbindung der Formel (I) enthaltenden Anstrichzusammensetzung um einen Decklack handelt, der als zusätzliches Lichtschutzmittel eine Verbindung aus der Reihe der Benzotriazole und/oder Hydroxyphenyltriazine enthält.

**9.** Schutzanstrichzusammensetzung für Holz, **dadurch gekennzeichnet, dass** die Anstrichzusammensetzung mindestens ein Benzophenon der Formel I

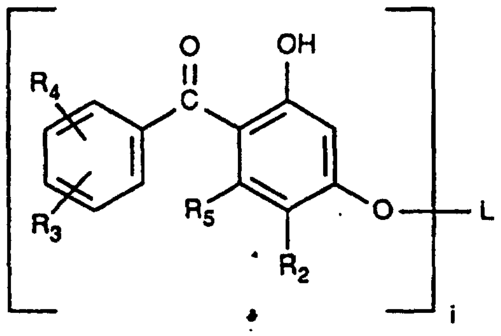

(I),

worin

i eine Zahl von eins bis vier,
L, wenn i eins ist, H, $C_1$-$C_{20}$-Alkyl, mit ein oder zwei -OH, mit Carboxyl, mit $C_1$-$C_{12}$-Alkoxy, mit Phenoxy, mit $C_2$-$C_{20}$-Alkylcarbonyl, mit $C_3$-$C_{20}$-Alkenylcarbonyl, mit $C_2$-$C_{20}$-Alkanoyloxy, mit $C_3$-$C_{20}$-Alkenoyloxy oder mit Phenyl substituiertes $C_2$-$C_{20}$-Alkyl, Glycidyl, mit ein bis sechs O-Atomen unterbrochenes oder mit ein oder zwei Carbonyloxy- oder Oxycarbonyl-Gruppen unterbrochenes $C_4$-$C_{20}$-Alkyl, $C_2$-$C_{18}$-Alkenyl, $C_2$-$C_{18}$-Alkanoyl, $C_3$-$C_{18}$-Alkenoyl, Benzoyl, mit ein oder zwei $C_1$-$C_4$-Alkyl-Gruppen substituiertes Benzoyl, gegebenenfalls substituiertes Phenyl oder Naphthyl oder eine Gruppe der Formel II, III, IV, V oder VI
$-(CH_2)_j-CH(OH)-(CH_2)_k-O-R_9$ (II) mit j, k = 0-4;
$-(CH_2)_m-COOR_9$ (III) mit m = 1-4;

wobei $R_9$ $C_1$-$C_{18}$-Alkyl oder Phenyl ist;

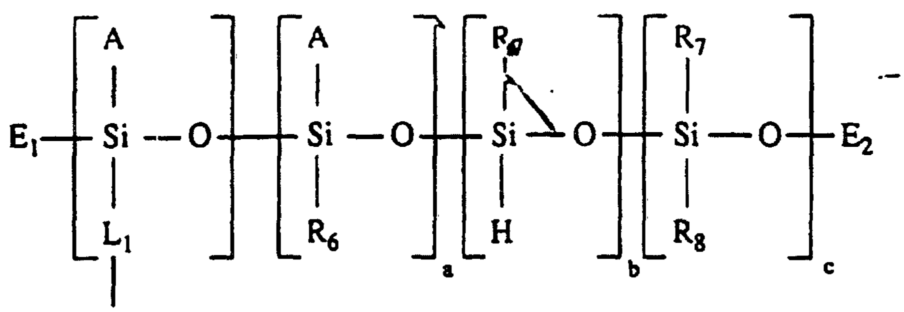

(IV);

wobei a = 0-50, b = 0-50 und c = 0-50 ist;
A $R_7$ oder $-O-Si(R_7)_3$;
$E_1$ OH, $C_1$-$C_{12}$-Alkyl, $C_1$-$C_4$-Alkoxy, Cyclohexyl, Phenyl oder $-O-Si(R_7)_3$;
$E_2$ H, $C_1$-$C_{12}$-Alkyl, Cyclohexyl, Phenyl, $-Si(R_7)_3$ oder $-Si(R_6)(R_7)_2$;
wobei $E_1$ und $E_2$ zusammen auch eine direkte Bindung bedeuten können;
$L_1$ eine direkte Bindung oder eine zweiwertige Gruppe der Formeln $-C_nH_{2n}-$, $-(CH_2)_n-O-$, $-CH_2CH(OH)CH_2-O-$ oder $-CH_2CH(OH)CH_2-O-(CH_2)_3-$, mit n = 1-4;
$R_6$ ein Rest der Formel

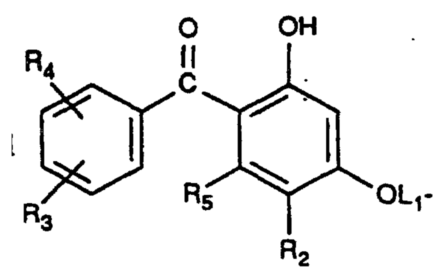

;

$R_7$-$C_1$-$C_{12}$-Alkyl, $C_1$-$C_4$-Alkoxy, Cyclohexyl oder Phenyl; und
$R_8$ $C_1$-$C_{18}$-Alkyl, $C_1$-$C_4$-Alkoxy, $C_5$-$C_7$-Cycloalkyl oder Phenyl ist;

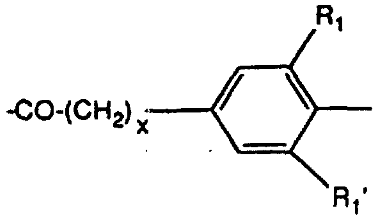 (V)

wobei x 0, 1 oder 2 ist;
$R_1$ $C_1$-$C_{12}$-Alkyl oder $C_5$-$C_8$-Cycloalkyl; und
$R_1$' sekundäres oder tertiäres $C_3$-$C_{12}$-Alkyl oder $C_5$-$C_8$-Cycloalkyl ist;

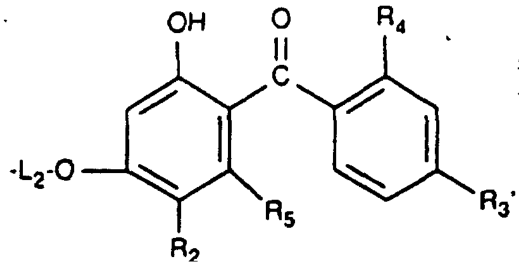 , (VI)

wobei $L_2$ die Bedeutung von L, wenn i zwei ist, hat; und
$R_3$' H oder $C_1$-$C_{18}$-Alkyl ist;

L, wenn i zwei ist, $C_1$-$C_{12}$-Alkylen, mit -OH, mit $C_1$-$C_8$-Alkoxy, mit $C_2$-$C_{20}$-Alkoxycarbonyl, mit $C_2$-$C_{20}$-Alkanoyloxy, mit $C_3$-$C_{20}$-Alkenoyloxy oder mit einer Silyl-Gruppe der Formel (IV) substituiertes $C_3$-$C_{12}$-Alkylen, mit ein bis sechs O-Atomen unterbrochenes oder mit ein oder zwei Carbonyloxy- oder Oxycarbonyl-Gruppen unterbrochenes $C_4$-$C_{20}$-Alkylen, o-Xylylen, m-Xylylen, p-Xylylen, iso-Phthaloyl, Phthaloyl, Terephthaloyl oder $\alpha,\omega$-$C_4$-$C_{12}$-Alkandioyl;
L, wenn i drei ist, $C_3$-$C_{12}$-Alkantriyl, $C_3$-$C_{12}$-Alkantrioyl, Trimellitoyl oder mit drei Carbonyloxy- oder Oxycarbonyl-Gruppen unterbrochenes $C_6$-$C_{20}$-Alkantriyl;
L, wenn i vier ist, $C_4$-$C_{16}$-Alkantetrayl, $C_4$-$C_{16}$-Alkantetroyl, Pyromellitoyl oder mit vier Carbonyloxy- oder Oxycarbonyl-Gruppen unterbrochenes $C_8$-$C_{24}$-Alkantetrayl;
$R_2$ H, $C_1$-$C_{18}$-Alkyl oder Cl;
$R_3$ -$SR_{10}$, -$SOR_{10}$ oder -$SO_2R_{10}$;
$R_4$ H, -OH, $C_1$-$C_{18}$-Alkyl oder $C_2$-$C_{18}$-Alkenyl;
$R_5$ H, -OL, mit L in der Bedeutung wenn i eins ist, oder Cl; und
$R_{10}$ $C_1$-$C_{20}$-Alkyl, mit -OH, mit $C_1$-$C_{12}$-Alkoxy, mit einer Silyl-Gruppe der Formel (IV), mit $C_2$-$C_{12}$-Alkanoyloxy, mit $C_3$-$C_{12}$-Alkenoyloxy oder mit Halogen substituiertes $C_2$-$C_{20}$-Alkyl, $C_3$-$C_{18}$-Alkenyl, $C_5$-$C_{12}$-Cycloalkyl, $C_7$-$C_{15}$-Phenylalkyl, $C_6$-$C_{10}$-Aryl, mit ein oder zwei $C_1$-$C_4$-Alkyl-Gruppen substituiertes $C_6$-$C_{10}$-Aryl oder 1,1,2,2-Tetrahydroperfluoro-$C_6$-$C_{16}$-alkyl;

bedeutet,
enthält.

**10.** Verwendung einer Verbindung der Formel I

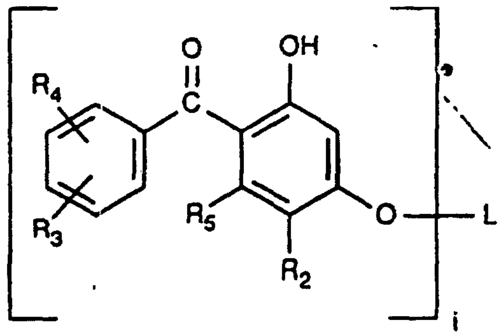

(I),

worin

i eine Zahl von eins bis vier,

L, wenn i eins ist, H, $C_1$-$C_{20}$-Alkyl, mit ein oder zwei -OH, mit Carboxyl, mit $C_1$-$C_{12}$-Alkoxy, mit Phenoxy, mit $C_2$-$C_{20}$-Alkylcarbonyl, mit $C_3$-$C_{20}$-Alkenylcarbonyl, mit $C_2$-$C_{20}$-Alkanoyloxy, mit $C_3$-$C_{20}$-Alkenoyloxy oder mit Phenyl substituiertes $C_2$-$C_{20}$-Alkyl, Glycidyl, mit ein bis sechs O-Atomen unterbrochenes oder mit ein oder zwei Carbonyloxy- oder Oxycarbonyl-Gruppen unterbrochenes $C_4$-$C_{20}$-Alkyl, $C_2$-$C_{18}$-Alkenyl, $C_2$-$C_{18}$-Alkanoyl, $C_3$-$C_{18}$-Alkenoyl, Benzoyl, mit ein oder zwei $C_1$-$C_4$-Alkyl-Gruppen substituiertes Benzoyl, gegebenenfalls substituiertes Phenyl oder Naphthyl oder eine Gruppe der Formel II, III, IV, V oder VI

$-(CH_2)_j-CH(OH)-(CH_2)_k-O-R_9$ (II) mit j, k =0-4;

$-(CH_2)_m-COOR_9$ (III) mit m =1-4;

wobei $R_9$ $C_1$-$C_{18}$-Alkyl oder Phenyl ist;

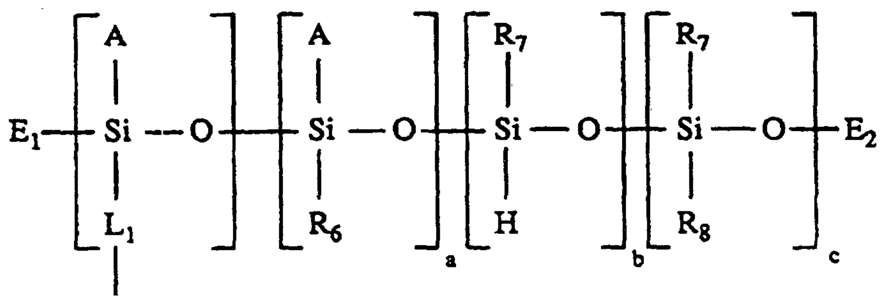

(IV);

wobei a = 0-50, b = 0-50 und c = 0-50 ist;

A $R_7$ oder $-O-Si(R_7)_3$;

$E_1$ OH, $C_1$-$C_{12}$-Alkyl, $C_1$-$C_4$-Alkoxy, Cyclohexyl, Phenyl oder $-O-Si(R_7)_3$;

$E_2$ H, $C_1$-$C_{12}$-Alkyl, Cyclohexyl, Phenyl, $-Si(R_7)_3$ oder $-Si(R_6)(R_7)_2$;

wobei $E_1$ und $E_2$ zusammen auch eine direkte Bindung bedeuten können;

$L_1$ eine direkte Bindung oder eine zweiwertige Gruppe der Formeln $-C_nH_{2n}-$, $-(CH_2)_n-O-$, $-CH_2CH(OH)CH_2-O-$ oder $-CH_2CH(OH)CH_2-O-(CH_2)_3-$, mit n = 1-4;

$R_6$ ein Rest der Forme

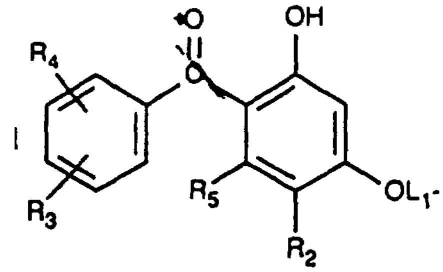

;

$R_7$ $C_1$-$C_{12}$-Alkyl, $C_1$-$C_4$-Alkoxy, Cyclohexyl oder Phenyl; und
$R_8$ $C_1$-$C_{18}$-Alkyl, $C_1$-$C_4$-Alkoxy, $C_5$-$C_7$-Cycloalkyl oder Phenyl ist;

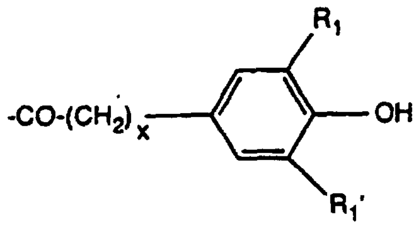
(V)

wobei x 0, 1 oder 2 ist;
$R_1$ $C_1$-$C_{12}$-Alkyl oder $C_5$-$C_8$-Cycloalkyl; und
$R_1$' sekundäres oder tertiäres $C_3$-$C_{12}$-Alkyl oder $C_5$-$C_8$-Cycloalkyl ist;

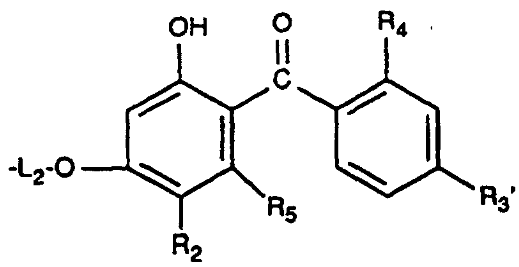
(VI)

wobei $L_2$ die Bedeutung von L, wenn i zwei ist, hat; und
$R_3$' H oder $C_1$-$C_{18}$-Alkyl ist;

L, wenn i zwei ist, $C_1$-$C_{12}$-Alkylen, mit -OH, mit $C_1$-$C_8$-Alkoxy, mit $C_2$-$C_{20}$-Alkoxycarbonyl, mit $C_2$-$C_{20}$-Alkanoyloxy, mit $C_3$-$C_{20}$-Alkenoyloxy oder mit einer Silyl-Gruppe der Formel (IV) substituiertes $C_3$-$C_{12}$-Alkylen, mit ein bis sechs O-Atomen unterbrochenes oder mit ein oder zwei Carbonyloxy- oder Oxycarbonyl-Gruppen unterbrochenes $C_4$-$C_{20}$-Alkylen, o-Xylylen, m-Xylylen, p-Xylylen, iso-Phthaloyl, Phthaloyl, Terephthaloyl oder $\alpha,\omega$-$C_4$-$C_{12}$-Alkandioyl;
L, wenn i drei ist, $C_3$-$C_{12}$-Alkantriyl, $C_3$-$C_{12}$-Alkantrioyl, Trimellitoyl oder mit drei Carbonyloxy- oder Oxycarbonyl-Gruppen unterbrochenes $C_6$-$C_{20}$-Alkantriyl;
L, wenn i vier ist, $C_4$-$C_{16}$-Alkantetrayl, $C_4$-$C_{16}$-Alkantetroyl, Pyromellitoyl oder mit vier Carbonyloxy- oder Oxycarbonyl-Gruppen unterbrochenes $C_8$-$C_{24}$-Alkantetrayl;
$R_2$ H, $C_1$-$C_{18}$-Alkyl oder Cl;
$R_3$ -$SR_{10}$, -$SOR_{10}$ oder -$SO_2R_{10}$;
$R_4$ H, -OH, $C_1$-$C_{18}$-Alkyl oder $C_2$-$C_{18}$-Alkenyl;
$R_5$ H, -OL, mit L in der Bedeutung wenn i eins ist, oder Cl; und
$R_{10}$ $C_1$-$C_{20}$-Alkyl, mit -OH, mit $C_1$-$C_{12}$-Alkoxy, mit einer Silyl-Gruppe der Formel (IV), mit $C_2$-$C_{12}$-Alkanoyloxy, mit $C_3$-$C_{12}$-Alkenoyloxy oder mit Halogen substituiertes $C_2$-$C_{20}$-Alkyl, $C_3$-$C_{18}$-Alkenyl, $C_5$-$C_{12}$-Cycloalkyl, $C_7$-$C_{15}$-Phenylalkyl, $C_6$-$C_{10}$-Aryl, mit ein oder zwei $C_1$-$C_4$-Alkyl-Gruppen substituiertes $C_6$-$C_{10}$-Aryl oder 1,1,2,2-Tetrahydroperfluoro-$C_6$-$C_{16}$-alkyl;

bedeutet,
zum Schützen von Holzoberflächen gegen Schädigung durch Licht.

**11.** Benzophenon der Formel I

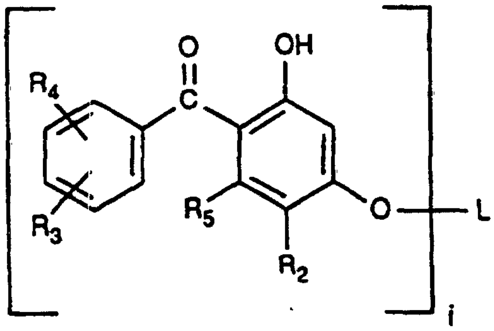

(I),

worin

i eine Zahl von eins bis vier,

L, wenn i eins ist, H, $C_1$-$C_{20}$-Alkyl, mit ein oder zwei -OH, mit Carboxyl, mit $C_1$-$C_{12}$-Alkoxy, mit Phenoxy, mit $C_2$-$C_{20}$-Alkylcarbonyl, mit $C_3$-$C_{20}$-Alkenylcarbonyl, mit $C_2$-$C_{20}$-Alkanoyloxy, mit $C_3$-$C_{20}$-Alkenoyloxy oder mit Phenyl substituiertes $C_2$-$C_{20}$-Alkyl, Glycidyl, mit ein bis sechs O-Atomen unterbrochenes oder mit ein oder zwei Carbonyloxy- oder Oxycarbonyl-Gruppen unterbrochenes $C_4$-$C_{20}$-Alkyl, $C_2$-$C_{18}$-Alkenyl, $C_2$-$C_{18}$-Alkanoyl, $C_3$-$C_{18}$-Alkenoyl, Benzoyl, mit ein oder zwei $C_1$-$C_4$-Alkyl-Gruppen substituiertes Benzoyl, gegebenenfalls substituiertes Phenyl oder Naphthyl oder eine Gruppe der Formel II, III, IV, V oder VI

$-(CH_2)_j-CH(OH)-(CH_2)_k-O-R_9$ (II) mit j, k = 0-4;

$-(CH_2)_m-COOR_9$ (III) mit m = 1-4;

wobei $R_9$ $C_1$-$C_{18}$-Alkyl oder Phenyl;

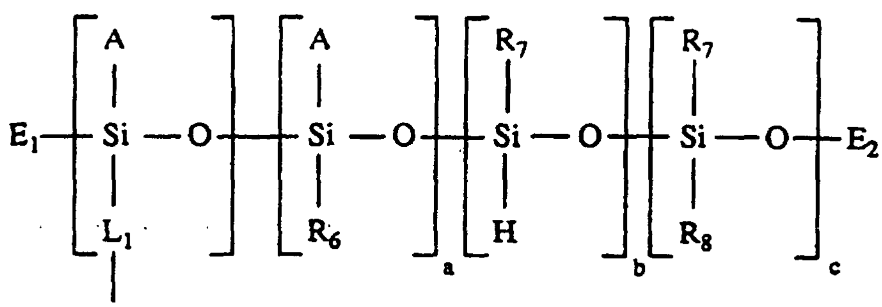

(IV);

wobei a =0-50, b = 0-50 und c = 0-50 ist;

A $R_7$ oder $-O-Si(R_7)_3$;

$E_1$ OH, $C_1$-$C_{12}$-Alkyl, $C_1$-$C_4$-Alkoxy, Cyclohexyl, Phenyl oder $-O-Si(R_7)_3$;

$E_2$ H, $C_1$-$C_{12}$-Alkyl, Cyclohexyl, Phenyl, $-Si(R_7)_3$ oder $-Si(R_6)(R_7)_2$;

wobei $E_1$ und $E_2$ zusammen auch eine direkte Bindung bedeuten können;

$L_1$ eine direkte Bindung oder eine zweiwertige Gruppe der Formeln $-C_nH_{2n}-$, $-(CH_2)_n-O-$, $-CH_2CH(OH)CH_2-O-$ oder $-CH_2CH(OH)CH_2-O-(CH_2)_3-$, mit n = 1-4;

$R_6$ ein Rest der Formel

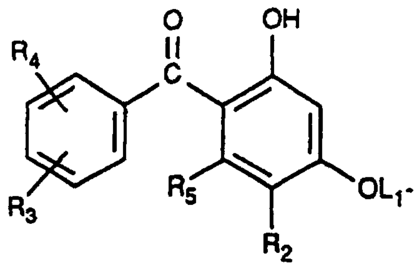

;

$R_7$ $C_1$-$C_{12}$-Alkyl, $C_1$-$C_4$-Alkoxy, Cyclohexyl oder Phenyl; und
$R_8$ $C_1$-$C_{18}$-Alkyl, $C_1$-$C_4$-Alkoxy, $C_5$-$C_7$-Cycloalkyl oder Phenyl ist;

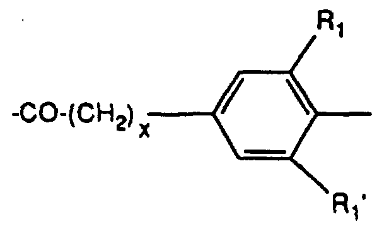

(V)

wobei x 0, 1 oder 2 ist;
$R_1$ $C_1$-$C_{12}$-Alkyl oder $C_5$-$C_8$-Cycloalkyl; und
$R_1$' sekundäres oder tertiäres $C_3$-$C_{12}$-Alkyl oder $C_5$-$C_8$-Cycloalkyl ist;

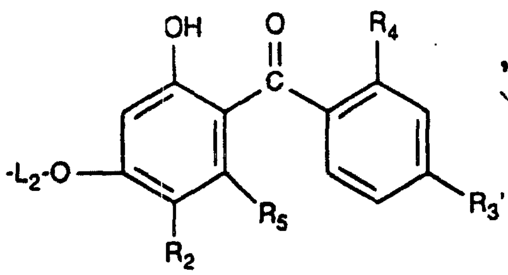

, (VI)

wobei $L_2$ die Bedeutung von L, wenn i zwei ist, hat; und
$R_3$' H oder $C_1$-$C_{18}$-Alkyl ist;

L, wenn i zwei ist, $C_1$-$C_{12}$-Alkylen, mit -OH, mit $C_1$-$C_8$-Alkoxy, mit $C_2$-$C_{20}$-Alkoxycarbonyl, mit $C_2$-$C_{20}$-Alkanoyloxy, mit $C_3$-$C_{20}$-Alkenoyloxy oder mit einer Silyl-Gruppe der Formel (IV) substituiertes $C_3$-$C_{12}$-Alkylen, mit ein bis sechs O-Atomen unterbrochenes oder mit ein oder zwei Carbonyloxy- oder Oxycarbonyl-Gruppen unterbrochenes $C_4$-$C_{20}$-Alkylen, o-Xylylen, m-Xylylen, p-Xylylen, iso-Phthaloyl, Phthaloyl, Terephthaloyl oder $\alpha,\omega$-$C_4$-$C_{12}$-Alkandioyl;
L, wenn i drei ist, $C_3$-$C_{12}$-Alkantriyl, $C_3$-$C_{12}$-Alkantrioyl, Trimellitoyl oder mit drei Carbonyloxy- oder Oxycarbonyl-Gruppen unterbrochenes $C_6$-$C_{20}$-Alkantriyl;
L, wenn i vier ist, $C_4$-$C_{16}$-Alkantetrayl, $C_4$-$C_{16}$-Alkantetroyl, Pyromellitoyl oder mit vier Carbonyloxy- oder Oxycarbonyl-Gruppen unterbrochenes $C_8$-$C_{24}$-Alkantetrayl;
$R_2$ H, $C_1$-$C_{18}$-Alkyl oder Cl;
$R_3$ -$SOR_{10}$ oder -$SO_2R_{10}$;
$R_4$ H, -OH, $C_1$-$C_{18}$-Alkyl oder $C_2$-$C_{18}$-Alkenyl;
$R_5$ H, -OL, mit L in der Bedeutung wenn i eins ist, oder Cl; und
$R_{10}$ $C_1$-$C_{20}$-Alkyl, mit -OH, mit $C_1$-$C_{12}$-Alkoxy, mit einer Silyl-Gruppe der Formel (IV), mit $C_2$-$C_{12}$-Alkanoyloxy, mit $C_3$-$C_{12}$-Alkenoyloxy oder mit Halogen substituiertes $C_2$-$C_{20}$-Alkyl, $C_3$-$C_{18}$-Alkenyl, $C_5$-$C_{12}$-Cycloalkyl, $C_7$-$C_{15}$-Phenylalkyl, $C_6$-$C_{10}$-Aryl, mit ein oder zwei $C_1$-$C_4$-Alkyl-Gruppen substituiertes $C_6$-$C_{10}$-Aryl oder 1,1,2,2-Tetrahydroperfluoro-$C_6$-$C_{16}$-alkyl;

bedeutet.

## Claims

1. A method of protecting a surface of wood against damage by light by treatment with a coating composition comprising a benzophenone of the formula I

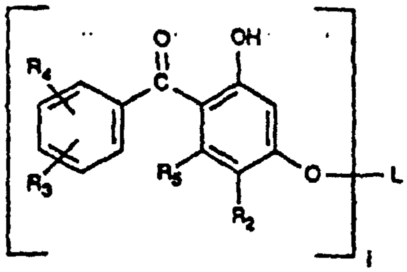

(I),

in which

i is a number from one to four;

L if i is one is H, $C_1$-$C_{20}$alkyl, $C_2$-$C_{20}$alkyl which is substituted by one or two -OH, by carboxyl, by $C_1$-$C_{12}$alkoxy, by phenoxy, by $C_2$-$C_{20}$alkylcarbonyl, by $C_3$-$C_{20}$alkenylcarbonyl, by $C_2$-$C_{20}$alkanoyloxy, by $C_3$-$C_{20}$alkenoyloxy or by phenyl, glycidyl, $C_4$-$C_{20}$alkyl which is interrupted by one to six O atoms or by one or two carbonyloxy or oxycarbonyl groups, $C_2$-$C_{18}$alkenyl, $C_2$-$C_{18}$alkanoyl, $C_3$-$C_{18}$alkenoyl, benzoyl, benzoyl which is substituted by one or two $C_1$-$C_4$alkyl groups, substituted or unsubstituted phenyl or naphthyl, or a group of the formula II, III, IV, V or VI:
$-(CH_2)_j-CH(OH)-(CH_2)_k-O-R_9$(II) where j, k = 0-4;
$-(CH_2)_m-COOR_9$ (III) where m = 1-4;

in which $R_9$ is $C_1$-$C_{18}$alkyl or phenyl;

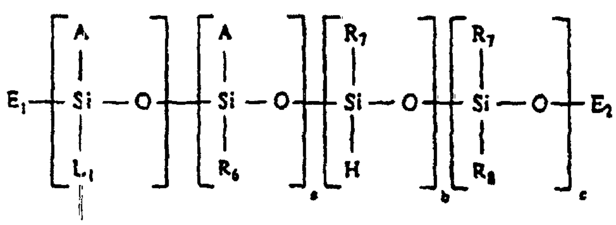

(IV);

in which a = 0-50, b = 0-50 and c = 0-50;
A is $R_7$ or $-O-Si(R_7)_3$;
$E_1$ is OH, $C_1$-$C_{12}$alkyl, $C_1$-$C_4$alkoxy, cyclohexyl, phenyl or $-O-Si(R_7)_3$;
$E_2$ is H, $C_1$-$C_{12}$alkyl, cyclohexyl, phenyl, $-Si(R_7)_3$ or $-Si(R_6)(R_7)_2$;
in which $E_1$ and $E_2$ together may also be a direct bond;
$L_1$ is a direct bond or a divalent group of the formula $-C_nH_{2n}-$, $-(CH_2)_n-O-$, $-CH_2CH(OH)CH_2-O-$ or $-CH_2CH(OH)CH_2-O-(CH_2)_3-$, where n = 1-4;
$R_6$ is a radical of the formula

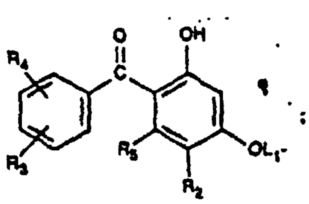

;

$R_7$ is $C_1$-$C_{12}$alkyl, $C_1$-$C_4$alkoxy, cyclohexyl or phenyl; and
$R_8$ is $C_1$-$C_{18}$alkyl, $C_1$-$C_4$alkoxy, $C_5$-$C_7$cycloalkyl or phenyl;

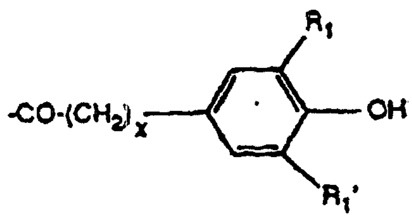

(V)

in which x is 0, 1 or 2;
$R_1$ is $C_1$-$C_{12}$alkyl or $C_5$-$C_8$cycloalkyl; and
$R_1$' is secondary or tertiary $C_3$-$C_{12}$alkyl or $C_5$-$C_8$cycloalkyl;

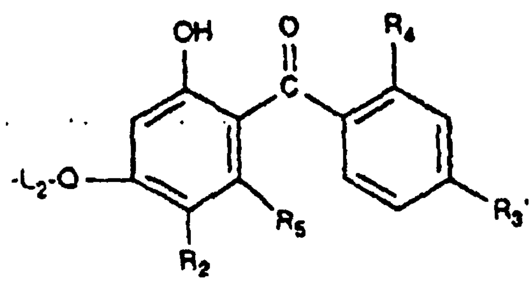

(VI)

in which $L_2$ is as defined for L if i is two; and $R_3$' is H or $C_1$-$C_{18}$alkyl;

L if i is two is $C_1$-$C_{12}$alkylene, $C_3$-$C_{12}$alkylene which is substituted by -OH, by $C_1$-$C_8$alkoxy, by $C_2$-$C_{20}$alkoxycarbonyl, by $C_2$-$C_{20}$alkanoyloxy, by $C_3$-$C_{20}$alkenoyloxy or by a silyl group of the formula (IV), $C_4$-$C_{20}$alkylene which is interrupted by one to six O atoms or by one or two carbonyloxy or oxycarbonyl groups, o-xylylene, m-xylylene, p-xylylene, isophthaloyl, phthaloyl, terephthaloyl or $\alpha,\omega$-$C_4$-$C_{12}$alkanedioyl;

L if i is three is $C_3$-$C_{12}$alkanetriyl, $C_3$-$C_{12}$alkanetrioyl, trimellitoyl or $C_6$-$C_{20}$alkanetriyl which is interrupted by three carbonyloxy or oxycarbonyl groups;

L if i is four is $C_4$-$C_{16}$alkanetetrayl, $C_4$-$C_{16}$alkanetetroyl, pyromellitoyl or $C_8$-$C_{24}$alkanetetrayl which is interrupted by four carbonyloxy or oxycarbonyl groups;

$R_2$ is H, $C_1$-$C_{18}$alkyl or Cl;
$R_3$ is $-SR_{10}$, $-SOR_{10}$ or $-SO_2R_{10}$;
$R_4$ is H, -OH, $C_1$-$C_{18}$alkyl or $C_2$-$C_{18}$alkenyl;
$R_5$ is H, -OL, where L is as defined for if i is one, or Cl; and
$R_{10}$ is $C_1$-$C_{20}$alkyl, $C_2$-$C_{20}$alkyl which is substituted by -OH, by $C_1$-$C_{12}$alkoxy, by a silyl group of the formula (IV), by $C_2$-$C_{12}$alkanoyloxy, by $C_3$-$C_{12}$alkenoyloxy or by halogen, $C_3$-$C_{18}$alkenyl, $C_5$-$C_{12}$cycloalkyl, $C_7$-$C_{15}$phenylalkyl, $C_6$-$C_{10}$aryl, $C_6$-$C_{10}$aryl which is substituted by one or two $C_1$-$C_4$alkyl groups, or 1,1,2,2-tetrahydroperfluoro-$C_6$-$C_{16}$alkyl.

**2.** A method according to claim 1, wherein the benzophenone is of the formula I in which

L is $C_1$-$C_{12}$alkyl;
$R_2$, $R_4$ and $R_5$ are H;
$R_3$ is $-SR_{10}$ or $-SO_2R_{10}$; and
$R_{10}$ is $C_1$-$C_{12}$alkyl or phenyl.

**3.** A method according to claim 1, wherein the coating composition comprising a compound of the formula (I) is a topcoat.

**4.** A method according to claim 1, wherein the coating composition comprising a compound of the formula (I) is a topcoat which is a clearcoat.

**5.** A method according to claim 1, wherein the coating composition comprising a compound of the formula (I) is a topcoat which contains a binder based on an alkyd resin.

**6.** A method according to claim 1, wherein the coating composition comprising a compound of the formula (I) is a topcoat which contains one or more further light stabilizers.

**7.** A method according to claim 1, wherein the coating composition comprising a compound of the formula (I) is a topcoat which contains a sterically hindered amine as additional light stabilizer.

**8.** A method according to claim 1, wherein the coating composition comprising a compound of the formula (I) is a topcoat which contains as additional light stabilizer a compound from the series of the benzotriazoles and/or hydroxyphenyltriazines.

**9.** A protective coating composition for wood, which comprises at least one benzophenone of the formula I

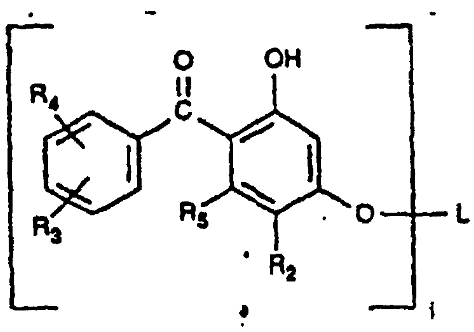

(I),

in which

i is a number from one to four;
L if i is one is H, $C_1$-$C_{20}$alkyl, $C_2$-$C_{20}$alkyl which is substituted by one or two -OH, by carboxyl, by $C_1$-$C_{12}$alkoxy, by phenoxy, by $C_2$-$C_{20}$alkylcarbonyl, by $C_3$-$C_{20}$alkenylcarbonyl, by $C_2$-$C_{20}$alkanoyloxy, by $C_3$-$C_{20}$alkenoyloxy or by phenyl, glycidyl, $C_4$-$C_{20}$alkyl which is interrupted by one to six O atoms or by one or two carbonyloxy or oxycarbonyl groups, $C_2$-$C_{18}$alkenyl, $C_2$-$C_{18}$alkanoyl, $C_3$-$C_{18}$alkenoyl, benzoyl, benzoyl which is substituted by one or two $C_1$-$C_4$alkyl groups, substituted or unsubstituted phenyl or naphthyl, or a group of the formula II, III, IV, V or VI:
$-(CH_2)_j-CH(OH)-(CH_2)_k-O-R_9$(II) where j, k = 0-4;
$-(CH_2)_m-COOR_9$ (III) where m = 1-4;

in which $R_9$ is $C_1$-$C_{18}$alkyl or phenyl;

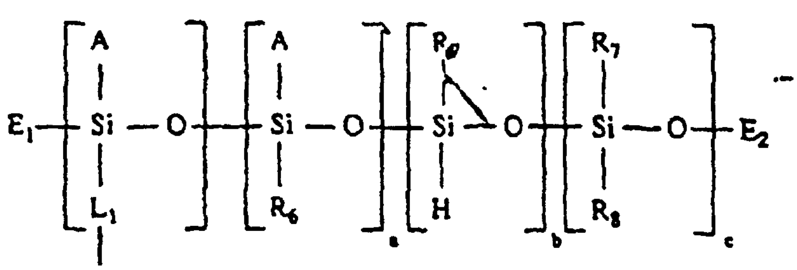

(IV);

in which a = 0-50, b = 0-50 and c = 0-50;
A is $R_7$ or $-O-Si(R_7)_3$;

$E_1$ is OH, $C_1$-$C_{12}$alkyl, $C_1$-$C_4$alkoxy, cyclohexyl, phenyl or -O-Si($R_7$)$_3$;
$E_2$ is H, $C_1$-$C_{12}$alkyl, cyclohexyl, phenyl, -Si($R_7$)$_3$ or - Si($R_6$) ($R_7$)$_2$;
in which $E_1$ and $E_2$ together may also be a direct bond;
$L_1$ is a direct bond or a divalent group of the formula $-C_nH_{2n}-$, $-(CH_2)_n-O-$, $-CH_2CH(OH)CH_2-O-$ or $-CH_2CH(OH)CH_2-O-(CH_2)_3-$, where n = 1-4;
$R_6$ is a radical of the formula

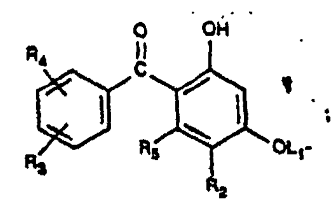

$R_7$ is $C_1$-$C_{12}$alkyl, $C_1$-$C_4$alkoxy, cyclohexyl or phenyl; and
$R_8$ is $C_1$-$C_{18}$alkyl, $C_1$-$C_4$alkoxy, $C_5$-$C_7$cycloalkyl or phenyl;

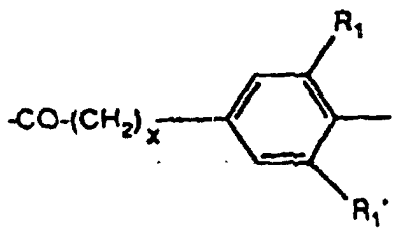 (V)

in which x is 0, 1 or 2;
$R_1$ is $C_1$-$C_{12}$alkyl or $C_5$-$C_8$cycloalkyl; and
$R_1'$ is secondary or tertiary $C_3$-$C_{12}$alkyl or $C_5$-$C_8$cycloalkyl;

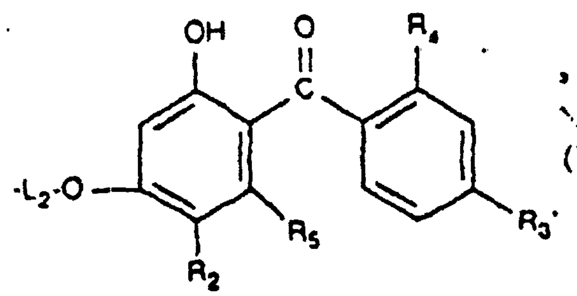 (VI)

in which $L_2$ is as defined for L if i is two; and
$R_3'$ is H or $C_1$-$C_{18}$alkyl ;

L if i is two is $C_1$-$C_{12}$alkylene, $C_3$-$C_{12}$alkylene which is substituted by -OH, by $C_1$-$C_8$alkoxy, by $C_2$-$C_{20}$alkoxycarbonyl, by $C_2$-$C_{20}$alkanoyloxy, by $C_3$-$C_{20}$alkenoyloxy or by a silyl group of the formula (IV), $C_4$-$C_{20}$alkylene which is interrupted by one to six O atoms or by one or two carbonyloxy or oxycarbonyl groups, o-xylylene, m-xylylene, p-xylylene, isophthaloyl, phthaloyl, terephthaloyl or $\alpha$, $\omega$-$C_4$-$C_{12}$alkanedioyl;
L if i is three is $C_3$-$C_{12}$alkanetriyl, $C_3$-$C_{12}$alkanetrioyl, trimellitoyl or $C_6$-$C_{20}$alkanetriyl which is interrupted by three carbonyloxy or oxycarbonyl groups;
L if i is four is $C_4$-$C_{16}$alkanetetrayl, $C_4$-$C_{16}$alkanetetroyl, pyromellitoyl or $C_8$-$C_{24}$alkanetetrayl which is interrupted by four carbonyloxy or oxycarbonyl groups;
$R_2$ is H, $C_1$-$C_{18}$alkyl or Cl;
$R_3$ is $-SR_{10}$, $-SOR_{10}$ or $-SO_2R_{10}$;
$R_4$ is H, -OH, $C_1$-$C_{18}$alkyl or $C_2$-$C_{18}$alkenyl;

$R_5$ is H, -OL, where L is as defined for if i is one, or Cl; and
$R_{10}$ is $C_1$-$C_{20}$alkyl, $C_2$-$C_{20}$alkyl which is substituted by -OH, by $C_1$-$C_{12}$alkoxy, by a silyl group of the formula (IV), by $C_2$-$C_{12}$alkanoyloxy, by $C_3$-$C_{12}$alkenoyloxy or by halogen, $C_3$-$C_{18}$alkenyl, $C_5$-$C_{12}$cycloalkyl, $C_7$-$C_{15}$phenylalkyl, $C_6$-$C_{10}$aryl, $C_6$-$C_{10}$aryl which is substituted by one or two $C_1$-$C_4$alkyl groups, or 1,1,2,2-tetrahydroperfluoro-$C_6$-$C_{16}$alkyl.

**10.** The use of a compound of the formula I

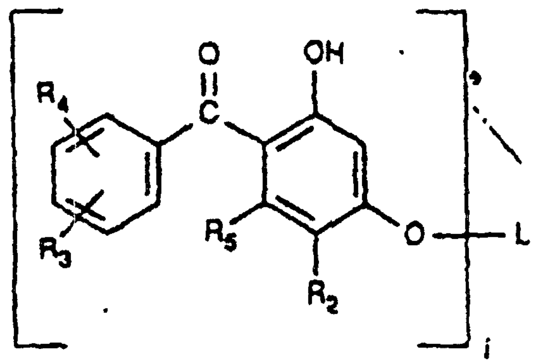

(I),

in which

i is a number from one to four;
L if i is one is H, $C_1$-$C_{20}$alkyl, $C_2$-$C_{20}$alkyl which is substituted by one or two -OH, by carboxyl, by $C_1$-$C_{12}$alkoxy, by phenoxy, by $C_2$-$C_{20}$alkylcarbonyl, by $C_3$-$C_{20}$alkenylcarbonyl, by $C_2$-$C_{20}$alkanoyloxy, by $C_3$-$C_{20}$alkenoyloxy or by phenyl, glycidyl, $C_4$-$C_{20}$alkyl which is interrupted by one to six O atoms or by one or two carbonyloxy or oxycarbonyl groups, $C_2$-$C_{18}$alkenyl, $C_2$-$C_{18}$alkanoyl, $C_3$-$C_{18}$alkenoyl, benzoyl, benzoyl which is substituted by one or two $C_1$-$C_4$alkyl groups, substituted or unsubstituted phenyl or naphthyl, or a group of the formula II, III, IV, V or VI:
$-(CH_2)_j-CH(OH)-(CH_2)_k-O-R_9$(II) where j, k = 0-4;
$-(CH_2)_m-COOR_9$ (III) where m = 1-4;

in which $R_9$ is $C_1$-$C_{18}$alkyl or phenyl;

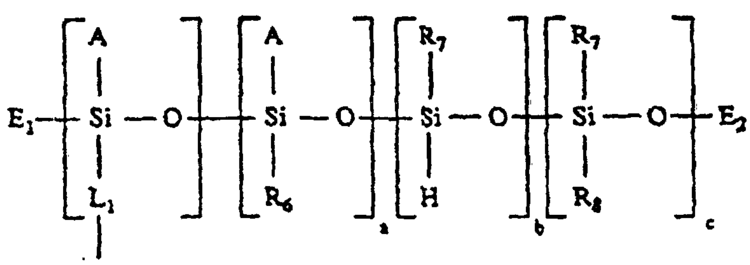

(IV);

in which a = 0-50, b = 0-50 and c = 0-50;
A is $R_7$ or $-O-Si(R_7)_3$;
$E_1$ is OH, $C_1$-$C_{12}$alkyl, $C_1$-$C_4$alkoxyl cyclohexyl, phenyl or $-O-Si(R_7)_3$;
$E_2$ is H, $C_1$-$C_{12}$alkyl, cyclohexyl, phenyl, $-Si(R_7)_3$ or $-Si(R_6)(R_7)_2$;
in which $E_1$ and $E_2$ together may also be a direct bond;
$L_1$ is a direct bond or a divalent group of the formula $-C_nH_{2n}-$, $-(CH_2)_n-O-$, $-CH_2CH(OH)CH_2-O-$ or $-CH_2CH(OH)CH_2-O-(CH_2)_3-$, where n = 1-4;
$R_6$ is a radical of the formula

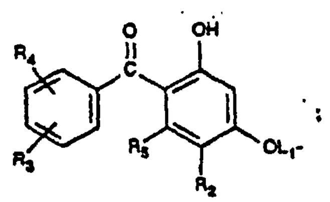

$R_7$ is $C_1$-$C_{12}$alkyl, $C_1$-$C_4$alkoxy, cyclohexyl or phenyl; and
$R_8$ is $C_1$-$C_{18}$alkyl, $C_1$-$C_4$alkoxy, $C_5$-$C_7$cycloalkyl or phenyl;

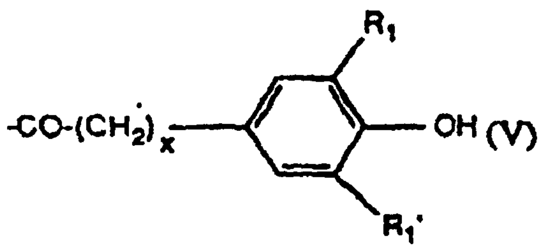

in which x is 0, 1 or 2;
$R_1$ is $C_1$-$C_{12}$alkyl or $C_5$-$C_8$cycloalkyl; and
$R_1'$ is secondary or tertiary $C_3$-$C_{12}$alkyl or $C_5$-$C_8$cycloalkyl;

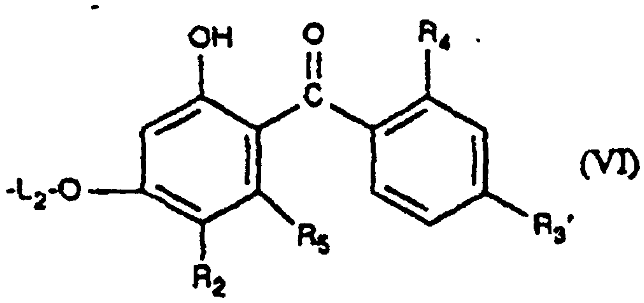

in which $L_2$ is as defined for L if i is two; and $R_3'$ is H or $C_1$-$C_{18}$alkyl;

L if i is two is $C_1$-$C_{12}$alkylene, $C_3$-$C_{12}$alkylene which is substituted by -OH, by $C_1$-$C_8$alkoxy, by $C_2$-$C_{20}$alkoxycarbonyl, by $C_2$-$C_{20}$alkanoyloxy, by $C_3$-$C_{20}$alkenoyloxy or by a silyl group of the formula (IV), $C_4$-$C_{20}$alkylene which is interrupted by one to six O atoms or by one or two carbonyloxy or oxycarbonyl groups, o-xylylene, m-xylylene, p-xylylene, isophthaloyl, phthaloyl, terephthaloyl or $\alpha,\omega$-$C_4$-$C_{12}$alkanedioyl;

L if i is three is $C_3$-$C_{12}$alkanetriyl, $C_3$-$C_{12}$alkanetrioyl, trimellitoyl or $C_6$-$C_{20}$alkanetriyl which is interrupted by three carbonyloxy or oxycarbonyl groups;

L if i is four is $C_4$-$C_{16}$alkanetetrayl, $C_4$-$C_{16}$alkanetetroyl, pyromellitoyl or $C_8$-$C_{24}$alkanetetrayl which is interrupted by four carbonyloxy or oxycarbonyl groups;

$R_2$ is H, $C_1$-$C_{18}$alkyl or Cl;

$R_3$ is $-SR_{10}$, $-SOR_{10}$ or $-SO_2R_{10}$;

$R_4$ is H, -OH, $C_1$-$C_{18}$alkyl or $C_2$-$C_{18}$alkenyl;

$R_5$ is H, -OL, where L is as defined for if i is one, or Cl; and

$R_{10}$ is $C_1$-$C_{20}$alkyl, $C_2$-$C_{20}$alkyl which is substituted by -OH, by $C_1$-$C_{12}$alkoxy, by a silyl group of the formula (IV), by $C_2$-$C_{12}$alkanoyloxy, by $C_3$-$C_{12}$alkenoyloxy or by halogen, $C_3$-$C_{18}$alkenyl, $C_5$-$C_{12}$cycloalkyl, $C_7$-$C_{15}$phenylalkyl, $C_6$-$C_{10}$aryl, $C_6$-$C_{10}$aryl which is substituted by one or two $C_1$-$C_4$alkyl groups, or 1,1,2,2-tetrahydroperfluoro-$C_6$-$C_{16}$alkyl,

for protecting surfaces of wood against damage by light.

**11.** A benzophenone of the formula I

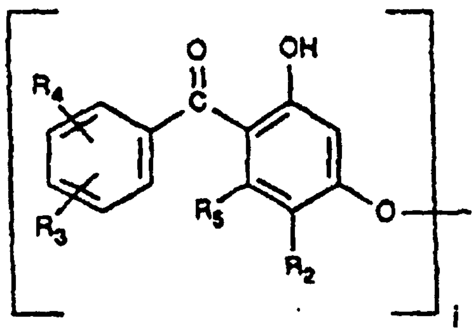

(I),

in which

i is a number from one to four;

L if i is one is H, $C_1$-$C_{20}$alkyl, $C_2$-$C_{20}$alkyl which is substituted by one or two -OH, by carboxyl, by $C_1$-$C_{12}$alkoxy, by phenoxy, by $C_2$-$C_{20}$alkylcarbonyl, by $C_3$-$C_{20}$alkenylcarbonyl, by $C_2$-$C_{20}$alkanoyloxy, by $C_3$-$C_{20}$alkenoyloxy or by phenyl, glycidyl, $C_4$-$C_{20}$alkyl which is interrupted by one to six O atoms or by one or two carbonyloxy or oxycarbonyl groups, $C_2$-$C_{18}$alkenyl, $C_2$-$C_{18}$alkanoyl, $C_3$-$C_{18}$alkenoyl, benzoyl, benzoyl which is substituted by one or two $C_1$-$C_4$alkyl groups, substituted or unsubstituted phenyl or naphthyl, or a group of the formula II, III, IV, V or VI:

$-(CH_2)_j-CH(OH)-(CH_2)_k-O-R_9$(II) where j, k = 0-4;

$-(CH_2)_m-COOR_9$ (III) where m = 1-4;

in which $R_9$ is $C_1$-$C_{18}$alkyl or phenyl;

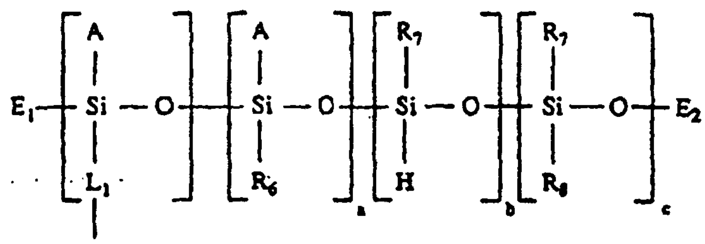

(IV);

in which a = 0-50, b = 0-50 and c = 0-50;

A is $R_7$ or $-O-Si(R_7)_3$;

$E_1$ is OH, $C_1$-$C_{12}$alkyl, $C_1$-$C_4$alkoxy, cyclohexyl, phenyl or $-O-Si(R_7)_3$;

$E_2$ is H, $C_1$-$C_{12}$alkyl, cyclohexyl, phenyl, $-Si(R_7)_3$ or $-Si(R_6)(R_7)_2$;

in which $E_1$ and $E_2$ together may also be a direct bond;

$R_6$ is a direct bond or a divalent group of the formula $-C_nH_{2n}-$, $-(CH_2)_n-O-$, $-CH_2CH(OH)CH_2-O-$ or $-CH_2CH(OH)CH_2-O-(CH_2)_3-$, where n = 1-4;

$R_6$ is a radical of the formula

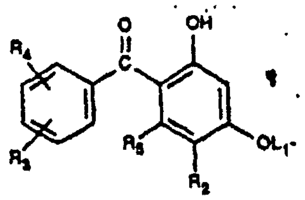

;

$R_7$ is $C_1$-$C_{12}$alkyl, $C_1$-$C_4$alkoxy, cyclohexyl or phenyl; and
$R_8$ is $C_1$-$C_{18}$alkyl, $C_1$-$C_4$alkoxy, $C_5$-$C_7$cycloalkyl or phenyl;

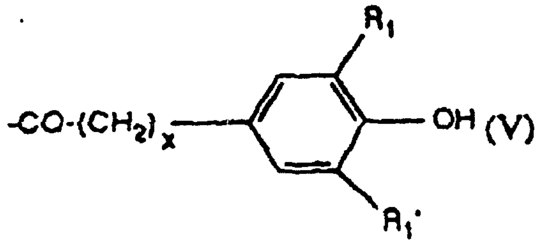

in which x is 0, 1 or 2;
$R_1$ is $C_1$-$C_{12}$alkyl or $C_5$-$C_8$cycloalkyl; and
$R_1$' is secondary or tertiary $C_3$-$C_{12}$alkyl or $C_5$-$C_8$cycloalkyl;

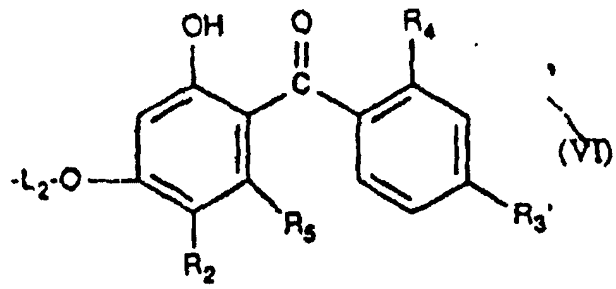

in which $L_2$ is as defined for L if i is two; and
$R_3$' is H or $C_1$-$C_{18}$alkyl;

L if i is two is $C_1$-$C_{12}$alkylene, $C_3$-$C_{12}$alkylene which is substituted by -OH, by $C_1$-$C_8$alkoxy, by $C_2$-$C_{20}$alkoxycarbonyl, by $C_2$-$C_{20}$alkanoyloxy, by $C_3$-$C_{20}$alkenoyloxy or by a silyl group of the formula (IV), $C_4$-$C_{20}$alkylene which is interrupted by one to six O atoms or by one or two carbonyloxy or oxycarbonyl groups, o-xylylene, m-xylylene, p-xylylene, isophthaloyl, phthaloyl, terephthaloyl or $\alpha,\omega$-$C_4$-$C_{12}$alkanedioyl;

L if i is three is $C_3$-$C_{12}$alkanetriyl, $C_3$-$C_{12}$alkanetrioyl, trimellitoyl or $C_6$-$C_{20}$alkanetriyl which is interrupted by three carbonyloxy or oxycarbonyl groups;

L if i is four is $C_4$-$C_{16}$alkanetetrayl, $C_4$-$C_{16}$alkanetetroyl, pyromellitoyl or $C_8$-$C_{24}$alkanetetrayl which is interrupted by four carbonyloxy or oxycarbonyl groups;

$R_2$ is H, $C_1$-$C_{18}$alkyl or Cl;
$R_3$ is -$SOR_{10}$ or -$SO_2R_{10}$;
$R_4$ is H, -OH, $C_1$-$C_{18}$alkyl or $C_2$-$C_{18}$alkenyl;
$R_5$ is H, -OL, where L is as defined for if i is one, or Cl; and
$R_{10}$ is $C_1$-$C_{20}$alkyl, $C_2$-$C_{20}$alkyl which is substituted by -OH, by $C_1$-$C_{12}$alkoxy, by a silyl group of the formula (IV), by $C_2$-$C_{12}$alkanoyloxy, by $C_3$-$C_{12}$alkenoyloxy or by halogen, $C_3$-$C_{18}$alkenyl, $C_5$-$C_{12}$cycloalkyl, $C_7$-$C_{15}$phenylalkyl, $C_6$-$C_{10}$aryl, $C_6$-$C_{10}$aryl which is substituted by one or two $C_1$-$C_4$alkyl groups, or 1,1,2,2-tetrahydroperfluoro-$C_6$-$C_{16}$alkyl.

## Revendications

**1.** Procédé pour la protection d'une surface de bois contre les dégradations induites par la lumière au moyen d'un traitement par une composition de peinture contenant une benzophénone de formule I

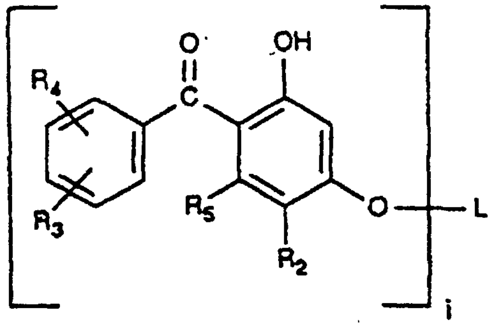

(I)

où

i est un nombre de 1 à 4 ;

L lorsque i vaut 1, représente un atome d'hydrogène, des groupes alkyle en $C_1$-$C_{20}$, alkyle en $C_2$-$C_{20}$ substitué par un ou deux substituants -OH, carboxyle, alkoxy en $C_1$-$C_{12}$, phénoxy, (alkyl en $C_2$-$C_{20}$) carbonyle, (alcényl en $C_3$-$C_{20}$) carbonyle, (alcanoyl en $C_2$-$C_{20}$)oxy, (alcénoyl en $C_3$-$C_{20}$)-oxy ou phényle, glycidyle, alkyle en $C_4$-$C_{20}$ interrompu par un à six atomes d'oxygène ou interrompu par un ou deux groupes carbonyloxy ou oxycarbonyle, alcényle en $C_2$-$C_{18}$, alcanoyle en $C_2$-$C_{18}$, alcénoyle en $C_3$-$C_{18}$, benzoyle, benzoyle substitué par un ou deux groupes alkyle en $C_1$-$C_4$, phényle ou naphtyle éventuellement substitués ou un groupe de formule II, III, IV, V ou VI
$-(CH_2)_j-CH(OH)-(CH_2)_k-O-R_9$ (II), où j, k = 0-4 ;
$-(CH_2)_m-COOR_9$ (III), où m = 1-4 ;
où $R_9$ représente des groupes alkyle en $C_1$-$C_{18}$ ou phényle ;

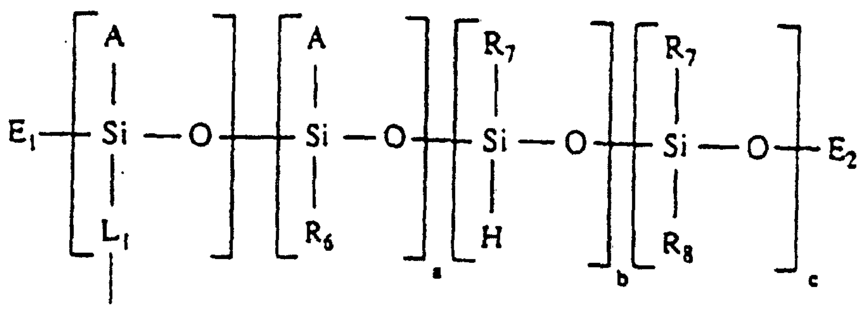

(IV)

où a = 0-50, b = 0-50 et c = 0-50 ;

A représente $R_7$ ou un groupe $-O-Si(R_7)_3$ ;

$E_1$ représente des groupes OH, alkyle en $C_1$-$C_{12}$, alkoxy en $C_1$-$C_4$, cyclohexyle, phényle ou $-O-Si(R_7)_3$ ;

$E_2$ représente un atome d'hydrogène, des groupes alkyle en $C_1$-$C_{12}$, cyclohexyle, phényle ou $-Si(R_7)_3$ ou $-Si(R_6)(R_7)_2$ ;

$E_1$ et $E_2$ pouvant également représenter conjointement une liaison directe ;

$L_1$ représente une liaison directe ou un groupe bivalent de formules $-C_nH_{2n}-$, $-(CH_2)_n-O-$, $-CH_2CH(OH)CH_2-O-$ ou $-CH_2-CH(OH)CH_2-O-(CH_2)_3-$, où n = 1-4 ;

$R_6$ représente un reste de formule

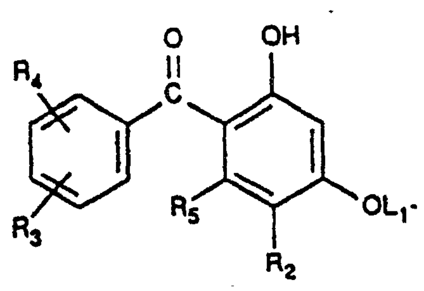 ;

$R_7$ représente des groupes alkyle en $C_1$-$C_{12}$, alkoxy en $C_1$-$C_4$, cyclohexyle ou phényle ; et

$R_8$ représente des groupes alkyle en $C_1$-$C_{18}$, alkoxy en $C_1$-$C_4$, cycloalkyle en $C_5$-$C_7$, ou phényle ; et

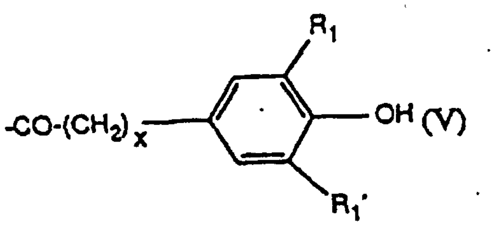

(V)

où

x vaut 0, 1 ou 2 ;

$R_1$ représente des groupes alkyle en $C_1$-$C_{12}$ ou cycloalkyle en $C_5$-$C_8$ ; et

$R_1$' représente un groupe alkyle en $C_3$-$C_{12}$ secondaire ou tertiaire ou un groupe cycloalkyle en $C_5$-$C_8$ ;

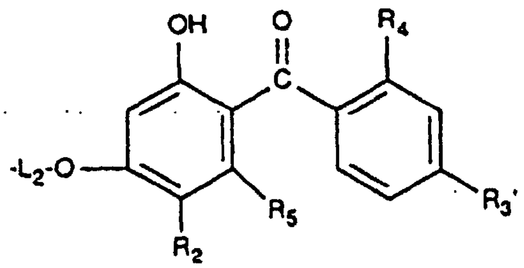

(VI)

$L_2$ possèdant la signification de L lorsque i vaut deux ; et

$R_3$' représente un atome d'hydrogène ou un groupe alkyle en $C_1$-$C_{18}$,

L lorsque i vaut 2, représente des groupes alkylène en $C_1$-$C_{12}$, alkylène en $C_3$-$C_{12}$ substitué par des substituants -OH, alkoxy en $C_1$-$C_8$, (alkoxy en $C_2$-$C_{20}$)carbonyle, (alcanoyl en $C_2$-$C_{20}$)-oxy, (alcénoyl en $C_3$-$C_{20}$)oxy ou un groupe silyle de formule (IV), alkylène en $C_4$-$C_{20}$ interrompu par un à six atomes d'oxygène ou par un ou deux groupes carbonyloxy ou oxycarbonyle, o-xylylène, m-xylylène, p-xylylène, iso-phtaloyle, phtaloyle, téréphtaloyle ou $\alpha,\omega$-(alcane en $C_4$-$C_{12}$)dioyle ;

L lorsque i vaut 3, représente des groupes (alcane en $C_3$-$C_{12}$)triyle, (alcane en $C_3$-$C_{12}$)trioyle, trimellitoyle ou (alcane en $C_6$-$C_{20}$)triyle interrompu par trois groupes carbonyloxy ou oxycarbonyle ;

L lorsque i vaut 4, représente des groupes (alcane en $C_4$-$C_{16}$) tétrayle, (alcane en $C_4$-$C_{16}$) tétroyle, pyromellitoyle ou (alcane en $C_8$-$C_{24}$) tétrayle interrompu par quatre groupes carbonyloxy ou oxycarbonyle ;

$R_2$ représente un atome d'hydrogène, des groupes alkyle en $C_1$-$C_{18}$ ou Cl ;

$R_3$ représente des groupes $-SR_{10}$, $-SOR_{10}$ ou $-SO_2R_{10}$ ;

$R_4$ représente un atome d'hydrogène, des groupes -OH, alkyle en $C_1$-$C_{18}$ ou alcényle en $C_2$-$C_{18}$ ;

$R_5$ représente un atome d'hydrogène, un groupe -OL, L ayant la signification donnée lorsque i vaut 1, ou un atome de Cl ; et

$R_{10}$ représente des groupes alkyle en $C_1$-$C_{20}$, alkyle en $C_2$-$C_{20}$ substitué par des substituants -OH, alkoxy en $C_1$-$C_{12}$, par un groupe silyle de formule (IV), par des substituants (alcanoyl en $C_2$-$C_{12}$)oxy, (alcénoyl en $C_3$-$C_{12}$)-oxy ou halogène, des groupes alcényle en $C_3$-$C_{18}$, cycloalkyle en $C_5$-$C_{12}$, phénylalkyle en $C_7$-$C_{15}$, aryle en $C_6$-$C_{10}$, aryle en $C_6$-$C_{10}$ substitué par un ou deux groupes alkyle en $C_1$-$C_4$, ou 1,1,2,2-tétrahydroperfluoroalkyle en $C_6$-$C_{16}$.

**2.** Procédé selon la revendication 1, **caractérisé en ce que** la benzophénone répond à la formule I, où

L représente un groupe alkyle en $C_1$-$C_{12}$ ;

$R_2$, $R_4$ et $R_5$ représentent des atomes d'hydrogène ;

$R_3$ représente des groupes -$SR_{10}$ ou -$SO_2R_{10}$ ; et

$R_{10}$ représente des groupes alkyle en $C_1$-$C_{12}$ ou phényle.

**3.** Procédé selon la revendication 1, caractérisé en que, pour une composition de peinture contenant un composé de formule (I), il s'agit d'un vernis de finition.

**4.** Procédé selon la revendication 1, **caractérisé en ce que**, pour une composition de peinture contenant un composé de formule (I), il s'agit d'un vernis de finition, qui est un vernis clair.

**5.** Procédé selon la revendication 1, **caractérisé en ce que**, pour une composition de peinture contenant un composé de formule (I), il s'agit d'un vernis de finition, qui contient un liant à base d'une résine alkyde.

**6.** Procédé selon la revendication 1, **caractérisé en ce que**, pour une composition de peinture contenant un composé de formule (I), il s'agit d'un vernis de finition, qui contient un ou plusieurs agents photoprotecteurs supplémentaires.

**7.** Procédé selon la revendication 1, **caractérisé en ce que**, pour une composition de peinture contenant un composé de formule (I), il s'agit d'un vernis de finition qui contient, en tant qu'agent photoprotecteur supplémentaire, une amine à encombrement stérique.

**8.** Procédé selon la revendication 1, **caractérisé en ce que**, pour une composition de peinture contenant un composé de formule (I), il s'agit d'un vernis de finition qui contient, en tant qu'agent photoprotecteur supplémentaire, un composé de la série des benzotriazoles et/ou hydroxyphényltriazines.

**9.** Composition de peinture de protection pour bois, **caractérisé en ce que** la composition de peinture contient au moins une benzophénone de formule I

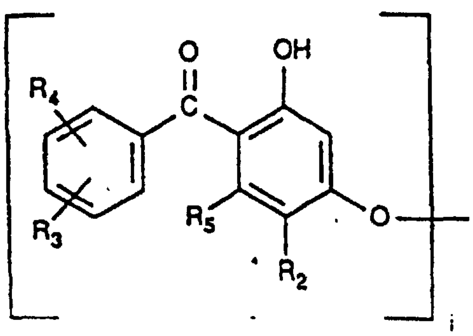

(I)

où

i est un nombre de 1 à 4 ;

L lorsque i vaut 1, représente un atome d'hydrogène, des groupes alkyle en $C_1$-$C_{20}$, alkyle en $C_2$-$C_{20}$ substitué par un ou deux substituants -OH, carboxyle, alkoxy en $C_1$-$C_{12}$, phénoxy, (alkyl en $C_2$-$C_{20}$)carbonyle, (alcényl en $C_3$-$C_{20}$)carbonyle, (alcanoyl en $C_2$-$C_{20}$)oxy, (alcénoyl en $C_3$-$C_{20}$)-oxy ou phényle, glycidyle, alkyle en

$C_4$-$C_{20}$ interrompu par un à six atomes d'oxygène ou interrompu par un ou deux groupes carbonyloxy ou oxycarbonyle, alcényle en $C_2$-$C_{18}$, alcanoyle en $C_2$-$C_{18}$, alcénoyle en $C_3$-$C_{18}$, benzoyle, benzoyle substitué par un ou deux groupes alkyle en $C_1$-$C_4$, phényle ou naphtyle éventuellement substitués ou un groupe de formule II, III, IV, V ou VI

$-(CH_2)_j-CH(OH)-(CH_2)_k-O-R_9$ (II), où j, k = 0-4 ;

$-(CH_2)_m-COOR_9$ (III), où m = 1-4 ; où $R_9$ représente des groupes alkyle en $C_1$-$C_{18}$ ou phényle ;

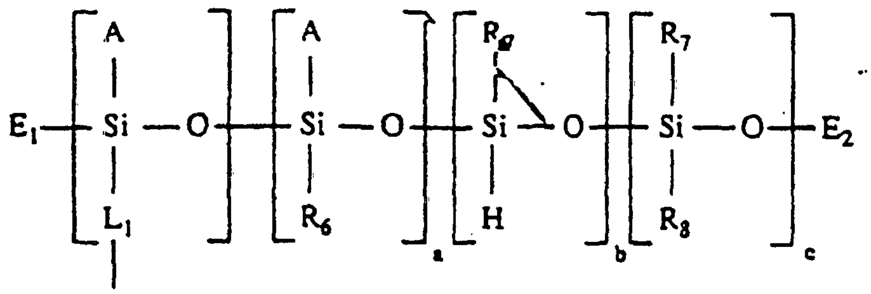

(IV),

ou a = 0-50, b = 0-50 et c = 0-50 ;

A représente $R_7$ ou un groupe $-O-Si(R_7)_3$ ;

$E_1$ représente des groupes OH, alkyle en $C_1$-$C_{12}$, alkoxy en $C_1$-$C_4$, cyclohexyle, phényle ou $-O-Si(R_7)_3$ ;

$E_2$ représente un atome d'hydrogène, des groupes alkyle en $C_1$-$C_{12}$, cyclohexyle, phényle ou $-Si(R_7)_3$ ou $-Si(R_6)(R_7)_2$ ;

$E_1$ et $E_2$ pouvant également représenter conjointement une liaison directe ;

$L_1$ représente une liaison directe ou un groupe bivalent de formules $-C_nH_{2n}-$, $-(CH_2)_n-O-$, $-CH_2CH(OH)CH_2-O-$ ou $-CH_2-CH(OH)CH_2-O-(CH_2)_3-$, avec n = 1-4 ;

$R_6$ représente un reste de formule

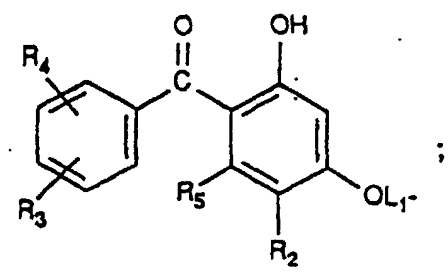

;

$R_7$ représente des groupes alkyle en $C_1$-$C_{12}$, alkoxy en $C_1$-$C_4$, cyclohexyle ou phényle ; et

$R_8$ représente des groupes alkyle en $C_1$-$C_{18}$, alkoxy en $C_1$-$C_4$, cycloalkyle en $C_5$-$C_7$, ou phényle ; et

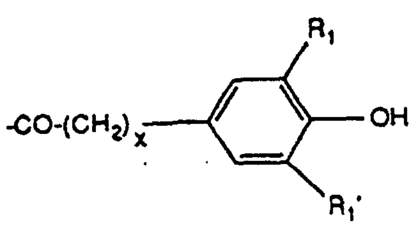

(V)

où

x vaut 0, 1 ou 2 ;

$R_1$ représente des groupes alkyle en $C_1$-$C_{12}$ ou cycloalkyle en $C_5$-$C_8$ ; et

$R_1'$ représente un groupe alkyle en $C_3$-$C_{12}$ secondaire ou tertiaire ou un groupe cycloalkyle en $C_5$-$C_8$ ;

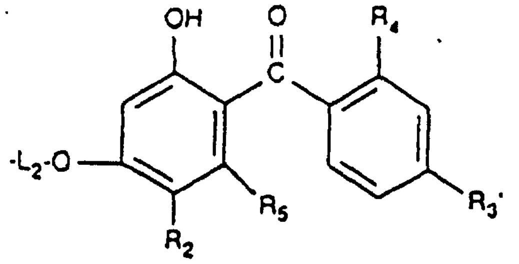

(VI)

$L_2$ possèdant la signification de L lorsque i vaut deux ; et
$R_3$' représente un atome d'hydrogène ou un groupe alkyle en $C_1$-$C_{18}$ ;

L lorsque i vaut 2, représente des groupes alkylène en $C_1$-$C_{12}$, alkylène en $C_3$-$C_{12}$ substitué par des substituants -OH, alkoxy en $C_1$-$C_8$, (alkoxy en $C_2$-$C_{20}$) carbonyle, (alcanoyl en $C_2$-$C_{20}$)-oxy, (alcénoyl en $C_3$-$C_{20}$) oxy ou un groupe silyle de formule (IV), alkylène en $C_4$-$C_{20}$ interrompu par un à six atomes d'oxygène ou par un ou deux groupes carbonyloxy ou oxycarbonyle, o-xylylène, m-xylylène, p-xylylène, iso-phtaloyle, phtaloyle, téréphtaloyle ou $\alpha,\omega$-(alcane en $C_4$-$C_{12}$) dioyle ;

L lorsque i vaut 3, représente des groupes (alcane en $C_3$-$C_{12}$) triyle, (alcane en $C_3$-$C_{12}$) trioyle, trimellitoyle ou (alcane en $C_6$-$C_{20}$)triyle interrompu par trois groupes carbonyloxy ou oxycarbonyle ;

L lorsque i vaut 4, représente des groupes (alcane en $C_4$-$C_{16}$) tétrayle, (alcane en $C_4$-$C_{16}$)tétroyle, pyromellitoyle ou (alcane en $C_8$-$C_{24}$)tétrayle interrompu par quatre groupes carbonyloxy ou oxycarbonyle ;

$R_2$ représente un atome d'hydrogène, des groupes alkyle en $C_1$-$C_{18}$ ou Cl ;

$R_3$ représente des groupes $-SR_{10}$, $-SOR_{10}$ ou $-SO_2R_{10}$ ;

$R_4$ représente un atome d'hydrogène, des groupes -OH, alkyle en $C_1$-$C_{18}$ ou alcényle en $C_2$-$C_{18}$ ;

$R_5$ représente un atome d'hydrogène, un groupe -OL, L ayant la signification donnée lorsque i vaut 1, ou un atome de Cl ; et

$R_{10}$ représente des groupes alkyle en $C_1$-$C_{20}$, alkyle en $C_2$-$C_{20}$ substitué par des substituants -OH, alkoxy en $C_1$-$C_{12}$, un groupe silyle de formule (IV), par des substituants (alcanoyl en $C_2$-$C_{12}$)-oxy, (alcénoyl en $C_3$-$C_{12}$)-oxy ou halogène, alcényle en $C_3$-$C_{18}$, cycloalkyle en $C_5$-$C_{12}$, phénylalkyle en $C_7$-$C_{15}$, aryle en $C_6$-$C_{10}$, aryle en $C_6$-$C_{10}$ substitué par un ou deux groupes alkyle en $C_1$-$C_4$, ou 1,1,2,2-tétrahydroperfluoroalkyle en $C_6$-$C_{16}$.

**10.** Utilisation d'un composé de formule I

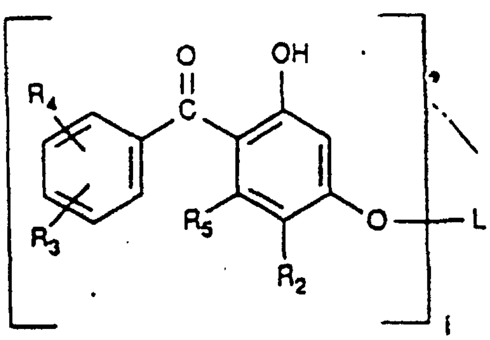

(I)

où

i est un nombre de 1 à 4 ;

L lorsque i vaut 1, représente un atome d'hydrogène, des groupes alkyle en $C_1$-$C_{20}$, alkyle en $C_2$-$C_{20}$ substitué par un ou deux substituants -OH, carboxyle, alkoxy en $C_1$-$C_{12}$, phénoxy, (alkyl en $C_2$-$C_{20}$) carbonyle, (alcényl en $C_3$-$C_{20}$)carbonyle, (alcanoyl en $C_2$-$C_{20}$)oxy, (alcénoyl en $C_3$-$C_{20}$)-oxy ou phényle, glycidyle, alkyle en $C_4$-$C_{20}$ interrompu par un à six atomes d'oxygène ou interrompu par un ou deux groupes carbonyloxy ou oxycarbonyle, alcényle en $C_2$-$C_{18}$, alcanoyle en $C_2$-$C_{18}$, alcénoyle en $C_3$-$C_{18}$ benzoyle, benzoyle substitué par un ou deux groupes alkyle en $C_1$-$C_4$, phényle ou naphtyle

éventuellement substitués ou un groupe de formule II, III, IV, V ou VI
$-(CH_2)_j-CH(OH)-(CH_2)_k-O-R_9$ (II), où j, k = 0-4 ;
$-(CH_2)_m-COOR_9$ (III), où m = 1-4 ;
où $R_9$ représente des groupes alkyle en $C_1$-$C_{18}$ ou phényle ;

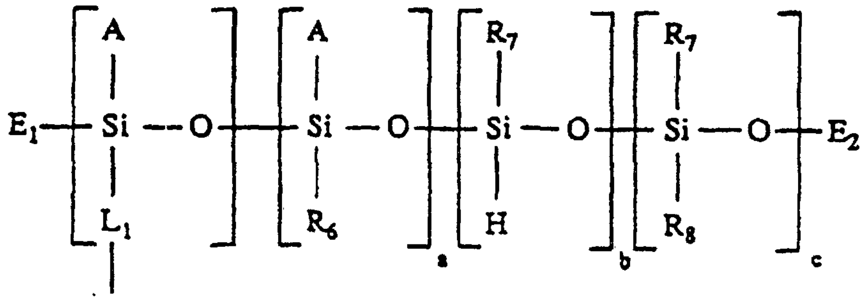 (IV) ;

où a = 0-50, b = 0-50 et c = 0-50 ;

A représente $R_7$ ou un groupe $-O-Si(R_7)_3$ ;
$E_1$ représente des groupes OH, alkyle en $C_1$-$C_{12}$, alkoxy en $C_1$-$C_4$, cyclohexyle, phényle ou $-O-Si(R_7)_3$ ;
$E_2$ représente un atome d'hydrogène, des groupes alkyle en $C_1$-$C_{12}$, cyclohexyle, phényle ou $-Si(R_7)_3$ ou $-Si(R_6)(R_7)_2$ ;
$E_1$ et $E_2$ pouvant également représenter conjointement une liaison directe ;
$L_1$ représente une liaison directe ou un groupe bivalent de formules $-C_nH_{2n}-$, $-(CH_2)_n-O-$, $-CH_2CH(OH)CH_2-O-$ ou $-CH_2-CH(OH)CH_2-O-(CH_2)_3-$, où n = 1-4 ;
$R_6$ représente un reste de formule

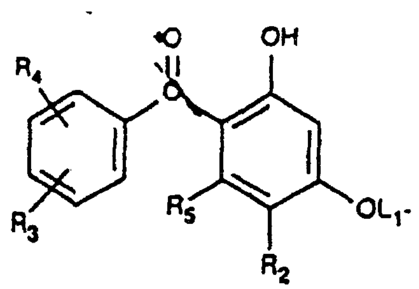 ;

$R_7$ représente des groupes alkyle en $C_1$-$C_{12}$, alkoxy en $C_1$-$C_4$, cyclohexyle ou phényle ; et
$R_8$ représente des groupes alkyle en $C_1$-$C_{18}$, alkoxy en $C_1$-$C_4$, cycloalkyle en $C_5$-$C_7$ ou phényle ; et

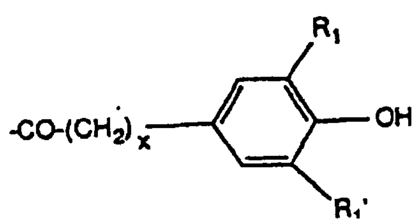 (V)

où

x vaut 0, 1 ou 2 ;
$R_1$ représente des groupes alkyle en $C_1$-$C_{12}$ ou cycloalkyle en $C_5$-$C_8$ ; et

$R_1'$ représente un groupe alkyle en $C_3$-$C_{12}$ secondaire ou tertiaire ou un groupe cycloalkyle en $C_5$-$C_8$ ;

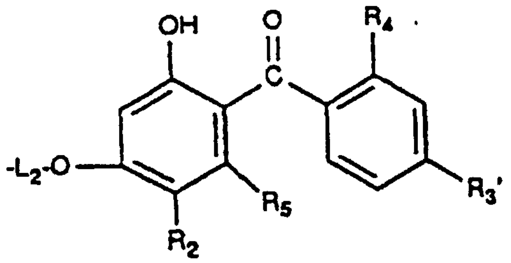

(VI)

$L_2$ possèdant la signification de L lorsque i vaut deux ; et
$R_3'$ représente un atome d'hydrogène ou un groupe alkyle en $C_1$-$C_{18}$,

L lorsque i vaut 2, représente des groupes alkylène en $C_1$-$C_{12}$, alkylène en $C_3$-$C_{12}$ substitué par des substituants -OH, alkoxy en $C_1$-$C_8$, (alkoxy en $C_2$-$C_{20}$)carbonyle, (alcanoyl en $C_2$-$C_{20}$)-oxy, (alcénoyl en $C_3$-$C_{20}$)oxy ou un groupe silyle de formule (IV), alkylène en $C_4$-$C_{20}$ interrompu par un à six atomes d'oxygène ou par un ou deux groupes carbonyloxy ou oxycarbonyle, o-xylylène, m-xylylène, p-xylylène, iso-phtaloyle, phtaloyle, téréphtaloyle ou $\alpha,\omega$-(alcane en $C_4$-$C_{12}$)dioyle ;

L lorsque i vaut 3, représente des groupes (alcane en $C_3$-$C_{12}$)triyle, (alcane en $C_3$-$C_{12}$)trioyle, trimellitoyle ou (alcane en $C_6$-$C_{20}$)triyle interrompu par trois groupes carbonyloxy ou oxycarbonyle ;

L lorsque i vaut 4, représente des groupes (alcane en $C_4$-$C_{16}$)tétrayle, (alcane en $C_4$-$C_{16}$)tétroyle, pyromellitoyle ou (alcane en $C_8$-$C_{24}$)tétrayle interrompu par quatre groupes carbonyloxy ou oxycarbonyle ;

$R_2$ représente un atome d'hydrogène, des groupes alkyle en $C_1$-$C_{18}$ ou Cl ;

$R_3$ représente des groupes $-SR_{10}$, $-SOR_{10}$ ou $-SO_2R_{10}$ ;

$R_4$ représente un atome d'hydrogène, des groupes -OH, alkyle en $C_1$-$C_{18}$ ou alcényle en $C_2$-$C_{18}$;

$R_5$ représente un atome d'hydrogène, un groupe -OL, L ayant la signification donnée lorsque i vaut 1, ou Cl ; et

$R_{10}$ représente un groupe alkyle en $C_1$-$C_{20}$, alkyle en $C_2$-$C_{20}$ substitué par des substituants -OH, alkoxy en $C_1$-$C_{12}$, un groupe silyle de formule (IV), (alcanoyl en $C_2$-$C_{12}$)oxy, (alcénoyl en $C_3$-$C_{12}$)-oxy ou halogène, alcényle en $C_3$-$C_{18}$, cycloalkyle en $C_5$-$C_{12}$, phénylalkyle en $C_7$-$C_{15}$, aryle en $C_6$-$C_{10}$, aryle en $C_6$-$C_{10}$ substitué par un ou deux groupes alkyle en $C_1$-$C_4$, ou 1,1,2,2-tétrahydroperfluoroalkyle en $C_6$-$C_{16}$ ;

pour la protection de surfaces de bois contre la dégradation induite par la lumière.

**11.** Benzophénone de formule I

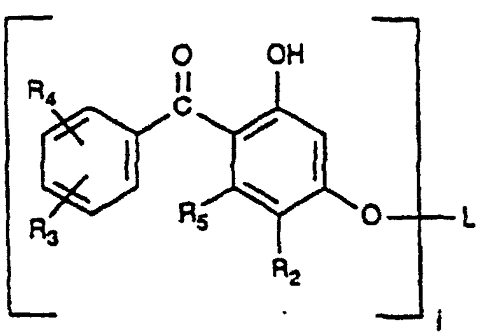

(I)

où

i est un nombre de 1 à 4 ;

L lorsque i vaut 1, représente un atome d'hydrogène, des groupes alkyle en $C_1$-$C_{20}$, alkyle en $C_2$-$C_{20}$ substitué par un ou deux groupes -OH, carboxyle, alkoxy en $C_1$-$C_{12}$, phénoxy, (alkyl en $C_2$-$C_{20}$) carbonyle, (alcényl en $C_3$-$C_{20}$) carbonyle, (alcanoyl en $C_2$-$C_{20}$)oxy, (alcénoyl en $C_3$-$C_{20}$)-oxy ou phényle, glycidyle, alkyle en $C_4$-$C_{20}$ interrompu par un à six atomes d'oxygène ou interrompu par un ou deux groupes carbonyloxy ou oxycarbonyle, alcénoyle en $C_3$-$C_{18}$, alcanoyle en $C_2$-$C_{18}$, alcényle en $C_2$-$C_{18}$, benzoyle, benzoyle substitué par un ou deux groupes alkyle en $C_1$-$C_4$, phényle ou naphtyle éventuellement substitué ou un groupe de formule II, III, IV, V ou VI

$-(CH_2)_j-CH(OH)-(CH_2)_k-O-R_9$ (II), où j, k = 0-4 ;

$-(CH_2)_m-COOR_9$ (III), où m = 1-4 ;

où $R_9$ représente des groupes alkyle en $C_1$-$C_{18}$ ou phényle ;

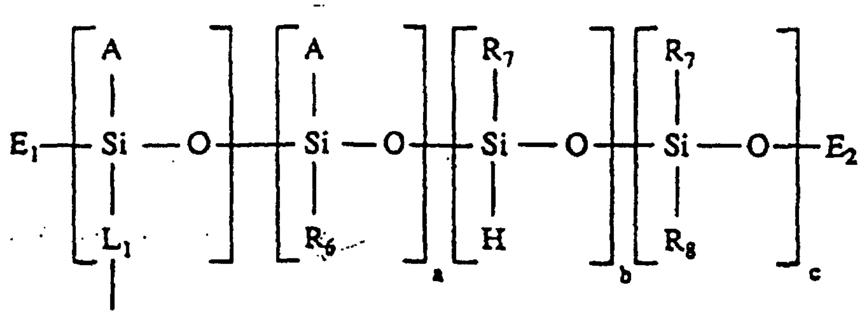
(IV) ;

où a = 0-50, b = 0-50 et c = 0-50 ;

A représente $R_7$ ou un groupe $-O-Si(R_7)_3$ ;

$E_1$ représente des groupes OH, alkyle en $C_1$-$C_{12}$, alkoxy en $C_1$-$C_4$, cyclohexyle, phényle ou $-O-Si(R_7)_3$ ;

$E_2$ représente des groupes H, alkyle en $C_1$-$C_{12}$, cyclohexyle, phényle ou $-Si(R_7)_3$ ou $-Si(R_6)(R_7)_2$ ;

$E_1$ et $E_2$ pouvant également représenter conjointement une liaison directe ;

$L_1$ représente une liaison directe ou un groupe bivalent de formules $-C_nH_{2n}-$, - $(CH_2)_n-O-$, $-CH_2CH(OH)CH_2-O-$ ou $-CH_2-CH(OH)CH_2-O-(CH_2)_3-$, avec n = 1-4 ;

$R_6$ représente un reste de formule

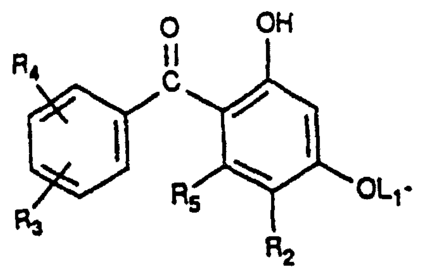
;

$R_7$ représente des groupes alkyle en $C_1$-$C_{12}$, alkoxy en $C_1$-$C_4$, cyclohexyle ou phényle ; et

$R_8$ représente des groupes alkyle en $C_1$-$C_{18}$, alkoxy en $C_1$-$C_4$, cycloalkyle en $C_5$-$C_7$, ou phényle ; et

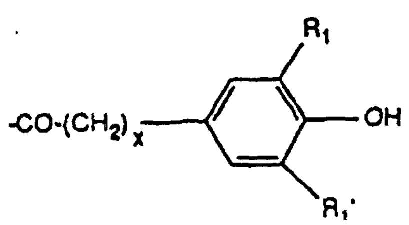
(V)

où

x vaut 0, 1 ou 2 ;
$R_1$ représente des groupes alkyle en $C_1$-$C_{12}$ ou cycloalkyle en $C_5$-$C_8$ ; et
$R_1$' représente un groupe alkyle en $C_3$-$C_{12}$ secondaire ou tertiaire ou un groupe cycloalkyle en $C_5$-$C_8$ ;

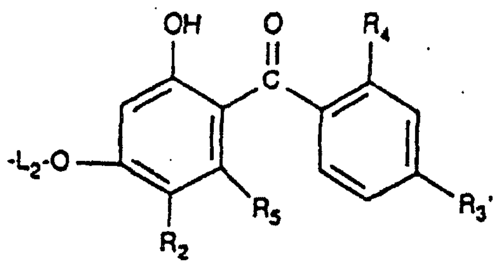

(VI)

$L_2$ possédant la signification de L lorsque i vaut deux ; et
$R_3$' représente un atome d'hydrogène ou un groupe alkyle en $C_1$-$C_{18}$,

L lorsque i vaut 2, représente des groupes alkylène en $C_1$-$C_{12}$, alkylène en $C_3$-$C_{12}$ substitué par des substituants -OH, alkoxy en $C_1$-$C_8$, (alkoxy en $C_2$-$C_{20}$)carbonyle, (alcanoyl en $C_2$-$C_{20}$)-oxy, (alcénoyl en $C_3$-$C_{20}$) oxy ou un groupe silyle de formule (IV), alkylène en $C_4$-$C_{20}$ interrompu par un à six atomes d'oxygène ou par un ou deux groupes carbonyloxy ou oxycarbonyle, o-xylylène, m-xylylène, p-xylylène, iso-phtaloyle, phtaloyle, téréphtaloyle ou $\alpha,\omega$-(alcane en $C_4$-$C_{12}$)dioyle ;
L lorsque i vaut 3, représente des groupes (alcane en $C_3$-$C_{12}$)triyle, (alcane en $C_3$-$C_{12}$)trioyle, trimellitoyle ou (alcane en $C_6$-$C_{20}$)triyle interrompu par trois groupes carbonyloxy ou oxycarbonyle ;
L lorsque i vaut 4, représente des groupes (alcane en $C_4$-$C_{16}$)tétrayle, (alcane en $C_4$-$C_{16}$)tétroyle, pyromellitoyle ou (alcane en $C_8$-$C_{24}$)tétrayle interrompu par quatre groupes carbonyloxy ou oxycarbonyle ;
$R_2$ représente un atome d'hydrogène ou des groupes alkyle en $C_1$-$C_{18}$ ou Cl ;
$R_3$ représente des groupes $-SOR_{10}$ ou $-SO_2R_{10}$ ;
$R_4$ représente un atome d'hydrogène ou des groupes -OH, alkyle en $C_1$-$C_{18}$ ou alcényle en $C_2$-$C_{18}$ ;
$R_5$ représente un atome d'hydrogène ou un groupe -OL, L ayant la signification donnée lorsque i vaut 1, ou Cl ; et
$R_{10}$ représente un groupe alkyle en $C_1$-$C_{20}$, alkyle en $C_2$-$C_{20}$ substitué par des substituants -OH, alkoxy en $C_1$-$C_{12}$, un groupe silyle de formule (IV), (alcanoyl en $C_2$-$C_{12}$)oxy, (alcénoyl en $C_3$-$C_{12}$)-oxy ou halogène, alcényle en $C_3$-$C_{18}$, cycloalkyle en $C_5$-$C_{12}$, phénylalkyle en $C_7$-$C_{15}$, aryle en $C_6$-$C_{10}$, aryle en $C_6$-$C_{10}$ substitué par un ou deux groupes alkyle en $C_1$-$C_4$, ou 1,1,2,2-tétrahydroperfluoroalkyle en $C_6$-$C_{16}$.